# EUROPEAN PATENT SPECIFICATION

(11) **EP 4 231 991 B1**
(45) Date of publication and mention of the grant of the patent: **09.12.2026**
(21) Application number: 21794178.0
(22) Date of filing: 21.10.2021
(51) Int. Cl.: A61K 8/34, A61K 8/44, A61K 8/46, A61Q 5/00, A61Q 19/00

(54) **OPACIFIER COMPOSITIONS**
TRÜBUNGSMITTELZUSAMMENSETZUNGEN
COMPOSITIONS OPACIFIANTES

(30) Priority: 23.10.2020 EP 20203607
(43) Date of publication of application: 30.08.2023
(73) Proprietor: Clariant International Ltd, 4132 Muttenz (CH)
(72) Inventor: STARKULLA, Gundula, North Salem, NY 10570 (US); CZECH, Katja, 65926 Frankfurt am Main (DE); PITARCH LOPEZ, Jesus, 65926 Frankfurt am Main (DE)
(74) Representative: Zusammenschluss Clariant
(86) International application number: PCT/EP2021/079298
(87) International publication number: WO 2022/084489

(56) References cited:
- US-A1- 2015 335 550
- US-A1- 2016 206 537
- DATABASE Mintel [online] GNPD; 1 October 2019 (2019-10-01), HUANYA COSMETICS: "Orchid Hidden Gem Fragrant Moisturising Body Wash", XP055794536, Database accession no. 6964147
- DATABASE Mintel [online] GNPD; 1 July 2016 (2016-07-01), OLEVA GROUP ENTERPRISE: "Firming Anti-Line/Wrinkle Cleanser", XP055794678, Database accession no. 4149115
- DATABASE Mintel [online] GNPD; 1 September 2020 (2020-09-01), AVN FOOTSTEPS: "Hair Cleanser", XP055794685, Database accession no. 8107967
- DATABASE Mintel [online] GNPD; 1 November 2020 (2020-11-01), EMMBROS OVERSEAS LIFESTYLE: "Shampoo", XP055794770, Database accession no. 8250285
- DATABASE Mintel [online] GNPD; 1 March 2018 (2018-03-01), PROYA COSMETICS: "Restoring Cleaning Foam", XP055794778, Database accession no. 5523805

## Description

The invention relates to an opacifier composition comprising (a) wax particles and (b) a sulfate-free anionic surfactant, its use as an opacifier in a cosmetic composition, as well as a cosmetic composition comprising the opacifier composition.

Opacifiers are additives that impart a rich, creamy, luxurious feel, an aesthetic appearance, and a higher visual viscosity to surfactant-based formulations like shampoos and body washes. They prevent light transmission and provide ideal brightness and whiteness to the end product. Product appearance is a key driver for consumer choice, and it is an important cue for essential product attributes (e.g. mildness, sensitivity).

Some of the most widely used opacifiers in cosmetic compositions today are based on styrene/acrylate copolymers.

However, there is a desire to replace such petroleum-derived opacifiers by more natural alternatives. There is a general desire for more sustainable, yet effective opacifier systems. In particular, there is a desire for opacifier systems with a high percentage of ingredients derived from renewable materials.

WO 2019/228975 discloses aqueous wax dispersions based on ethylene glycol fatty acid esters, which can be used as opacifiers in cosmetic compositions. These wax dispersions, however, contain fatty alcohol ether sulfates as surfactants. Sulfate-containing surfactants can be irritating to hair and/or skin.

Consumers have generally become more conscious about the ingredients used in cosmetic products. Among others, they increasingly desire sulfate-free ingredients.

It has now been found that compositions comprising wax particles and a sulfate-free anionic surfactant can be efficiently used as opacifiers in cosmetic compositions.

Accordingly, the invention relates to an opacifier composition comprising
(a) from 20 to 35 wt.-% of wax particles, based on the total weight of the opacifier composition; and
(b) from 2 to 12 wt.-% of a sulfate-free anionic surfactant, based on the total weight of the opacifier composition,

wherein the wax particles comprise mono- or di- C8-C20 esters of ethylene glycol, diethylene glycol or triethylene glycol, or rice bran wax, oxidized rice bran wax, rice bran wax ethyl esters, or mixtures thereof,
wherein the sulfate-free anionic surfactant is selected from acyl isethionates, acyl taurates, acyl glutamates, and mixtures thereof.

Advantageously, the sulfate-free anionic surfactants used in the opacifier compositions are mild and gentle to hair and/or skin. The opacifier compositions can be prepared using ingredients derived from renewable materials, which is beneficial from a sustainability perspective. The opacifier compositions can be prepared using biodegradable ingredients. Advantageously, the opacifier compositions enhance the appearance (in particular opacity), performance (for example cleansing and foam morphology), skin feel (for example sensory during use and after-feel) and skin condition (for example refatting effect) of surfactant containing cosmetic compositions, especially hair and/or skin cleansing compositions.

According to the invention, the opacifier composition comprises wax particles (a).

Preferred wax particles comprise mono- or di- C8-C20 esters of ethylene glycol, diethylene glycol or triethylene glycol, or mixtures thereof. More preferred wax particles comprise mono- or di- C12-C18 esters of ethylene glycol, diethylene glycol or triethylene glycol, or mixtures thereof.

More preferred wax particles comprise mono- or di- C8-C20 esters of ethylene glycol or diethylene glycol, or mixtures thereof. Even more preferred wax particles comprise mono- or di- C8-C20 esters of ethylene glycol, or mixtures thereof. Particularly preferred wax particles comprise mono- or di- C12-C18 esters of ethylene glycol, or mixtures thereof.

More preferred wax particles comprise di- C8-C20 esters of ethylene glycol, diethylene glycol or triethylene glycol, or mixtures thereof. Also preferred wax particles comprise mono- C8-C20 esters of ethylene glycol, diethylene glycol or triethylene glycol, or mixtures thereof.

More preferred wax particles comprise di- C8-C20 esters of ethylene glycol. Even more preferred wax particles comprise di- C12-C18 esters of ethylene glycol. Also preferred wax particles comprise mono- C8-C20 esters of ethylene glycol. Also preferred wax particles comprise mono- C12-C18 esters of ethylene glycol. More preferred wax particles comprise di- C8-C20 esters of ethylene glycol and mono- C8-C20 esters of ethylene glycol. Even more preferred wax particles comprise di- C12-C18 esters of ethylene glycol and mono- C12-C18 esters of ethylene glycol.

Preferred wax particles comprise ethylene glycol distearate, ethylene glycol monostearate, ethylene glycol dipalmitate, ethylene glycol monopalmitate, or mixtures thereof.

More preferred wax particles comprise ethylene glycol distearate, ethylene glycol dipalmitate, or mixtures thereof. Even more preferred wax particles comprise ethylene glycol distearate. Also even more preferred wax particles comprise ethylene glycol dipalmitate. Particularly preferred wax particles comprise ethylene glycol distearate and ethylene glycol dipalmitate.

Also preferred wax particles comprise ethylene glycol distearate, ethylene glycol monostearate, or mixtures thereof. More preferred wax particles comprise ethylene glycol distearate and ethylene glycol monostearate. Even more preferred wax particles comprise ethylene glycol distearate, ethylene glycol monostearate and ethylene glycol dipalmitate. Particularly preferred wax particles comprise ethylene glycol distearate, ethylene glycol monostearate, ethylene glycol dipalmitate and ethylene glycol monopalmitate.

In preferred embodiments, the wax particles comprise at least 50 wt.-%, preferably at least 60 wt.-%, more preferably at least 70 wt.-%, even more preferably at least 75 wt.-%, particularly preferably at least 80 wt.-% of di- C8-C20 esters of ethylene glycol, based on the total weight of the wax particles. Preferably, the di- C8-C20 esters of ethylene glycol are di- C12-C18 esters of ethylene glycol, more preferably ethylene glycol distearate and ethylene glycol dipalmitate, particularly preferably ethylene glycol distearate.

In preferred embodiments, the wax particles comprise up to 50 wt.-%, preferably up to 40 wt.-%, more preferably up to 30 wt.-%, even more preferably up to 25 wt.-%, particularly preferably up to 20 wt.-% of mono- C8-C20 esters of ethylene glycol, based on the total weight of the wax particles. Preferably, the mono- C8-C20 esters of ethylene glycol are mono- C12-C18 esters of ethylene glycol, more preferably ethylene glycol monostearate and ethylene glycol monopalmitate, particularly preferably ethylene glycol monostearate.

In preferred embodiments, the wax particles comprise mono- or di- C16 esters of ethylene glycol and mono- or di- C18 esters of ethylene glycol. Preferably, the weight ratio of mono- or di- C16 esters of ethylene glycol to mono- or di- C18 esters of ethylene glycol is in the range of from 2:1 to 1:20, more preferably from 1.5:1 to 1:15, even more preferably from 1.2:1 to 1:12, even more preferably from 1:1 to 1:10, even more preferably from 1:1 to 1:5, particularly preferably from 1:1 to 1:2. For example, the weight ratio of mono- or di- C16 esters of ethylene glycol to mono- or di- C18 esters of ethylene glycol is 2:1 or 1.5:1 or 1.2:1 or 1:1 or 1:2 or 1:5 or 1:10 or 1:15 or 1:20.

Preferred wax particles comprise a natural wax. Particularly preferred wax particles comprise rice bran wax.

Also preferred wax particles comprise a modified wax. More preferred wax particles comprise an oxidized wax, an esterified wax, or mixtures thereof. Also more preferred wax particles comprise a modified rice bran wax. Even more preferred wax particles comprise oxidized rice bran wax or rice bran wax ethyl esters, or mixtures thereof. Particularly preferred wax particles comprise oxidized rice bran wax. Oxidized rice bran wax is, e.g., commercially available from Clariant (Licocare^{®} RBW 102 VITA).

The wax particles comprise mono- or di- C8-C20 esters of ethylene glycol, diethylene glycol or triethylene glycol, or rice bran wax, oxidized rice bran wax, rice bran wax ethyl esters, or mixtures thereof.

In preferred embodiments, the wax particles comprise rice bran wax, oxidized rice bran wax, rice bran wax ethyl esters, or mixtures thereof.

The opacifier composition of the invention comprises from 20 to 35 wt.-%, particularly preferably from 25 to 30 wt.-% of wax particles (a), based on the total weight of the opacifier composition.

According to the invention, the opacifier composition comprises a sulfate-free anionic surfactant (b). The term "sulfate-free anionic surfactant" as used herein refers to an anionic surfactant which does not bear a sulfate group or a group -OSO₃H.

The opacifier composition of the invention may comprise one or more sulfate-free anionic surfactants. Preferably, the opacifier composition of the invention comprises 1 to 3 sulfate-free anionic surfactants, more preferably 1 or 2 sulfate-free anionic surfactants, particularly preferably 1 sulfate-free anionic surfactant.

The sulfate-free anionic surfactant is selected from acyl isethionates, acyl taurates, acyl glutamates, and mixtures thereof. Even more preferably, the sulfate-free anionic surfactant is selected from acyl isethionates, acyl taurates, and mixtures thereof. Particularly preferably, the sulfate-free anionic surfactant is selected from acyl isethionates. Also particularly preferably, the sulfate-free anionic surfactant is selected from acyl taurates. Also particularly preferably, the sulfate-free anionic surfactant is selected from acyl glutamates.

The sulfate-free anionic surfactants can, for example, be used in the form of their water-soluble or water-dispersible salts. Preferred salts are lithium, sodium, potassium, magnesium, calcium, aluminium, ammonium, monoalkylammonium, dialkylammonium, trialkylammonium or tetraalkylammonium salts, or mixtures thereof. More preferred salts are sodium, potassium or ammonium salts, or mixtures thereof. Particularly preferred salts are sodium salts.

Examples of more preferred sulfate-free anionic surfactants are sodium lauroyl isethionate, sodium cocoyl isethionate, sodium methyl cocoyl taurate, sodium cocoyl glutamate, or mixtures thereof. Examples of even more preferred sulfate-free anionic surfactants are sodium cocoyl isethionate, sodium methyl cocoyl taurate, sodium cocoyl glutamate, or mixtures thereof. Examples of particularly preferred sulfate-free anionic surfactants are sodium cocoyl isethionate, sodium methyl cocoyl taurate, or mixtures thereof.

In at least one embodiment, the sulfate-free anionic surfactant is selected from acyl isethionates, preferably acyl isethionates of formula (W): 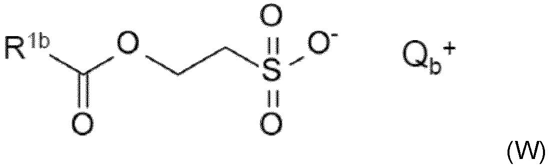 wherein
R^{1b} is a linear or branched, saturated alkyl group having 6 to 30, preferably 8 to 22, more preferably 8 to 18 carbon atoms or is a linear or branched, mono- or polyunsaturated alkenyl group having 6 to 30, preferably 8 to 22, more preferably 12 to 18 carbon atoms, and
Q_{b}⁺ is a cation.

Preferably, Q_{b}⁺ is selected from the group consisting of Li⁺, Na⁺, K⁺, Mg⁺⁺, Ca⁺⁺, Al⁺⁺⁺, NH₄⁺, a monoalkylammonium ion, a dialkylammonium ion, a trialkylammonium ion and a tetraalkylammonium ion, or mixtures thereof. More preferably, Q_{b}⁺ is sodium. Particularly preferably, R^{1b} is C12 alkyl or C14 alkyl. Also particularly preferably, R^{1b} is C16 alkyl or C18 alkyl.

A particularly preferred acyl isethionate is sodium cocoyl isethionate. Sodium cocoyl isethionate is, e.g., commercially available from Clariant (Hostapon^{®} SCI- 65 C, Hostapon^{®} SCI- 85 C).

Acyl isethionates and in particular acyl isethionates of formula (W) are beneficial because they are particularly mild (also compared to sodium laureth sulfate).

In at least one embodiment, the sulfate-free anionic surfactant is selected from acyl taurates, preferably acyl taurates of formula (X): 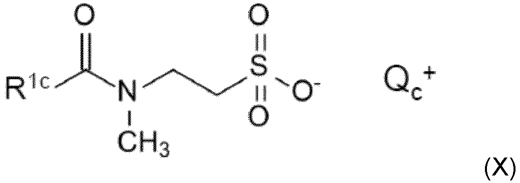 wherein
R^{1c} is a linear or branched, saturated alkyl group having 6 to 30, preferably 8 to 22, more preferably 8 to 18 carbon atoms or is a linear or branched, mono- or polyunsaturated alkenyl group having 6 to 30, preferably 8 to 22, more preferably 12 to 18 carbon atoms, and
Q_{c}⁺ is a cation.

Preferably, Q_{c}⁺ is selected from the group consisting of Li⁺, Na⁺, K⁺, Mg⁺⁺, Ca⁺⁺, Al⁺⁺⁺, NH₄⁺, a monoalkylammonium ion, a dialkylammonium ion, a trialkylammonium ion and a tetraalkylammonium ion, or mixtures thereof. More preferably, Q_{c}⁺ is sodium. Particularly preferably, R^{1c} is C12 alkyl or C14 alkyl. Also particularly preferably, R^{1c} is C16 alkyl or C18 alkyl.

A particularly preferred acyl taurate is sodium methyl cocoyl taurate. Sodium methyl cocoyl taurate is, e.g., commercially available from Clariant (Hostapon^{®} CT paste).

Acyl taurates and in particular acyl taurates of formula (X) are beneficial because they are particularly mild (also compared to sodium laureth sulfate) and stable over a broad pH range.

In at least one embodiment, the sulfate-free anionic surfactant is selected from acyl glutamates, preferably acyl glutamates of formula (Z) or salts thereof: 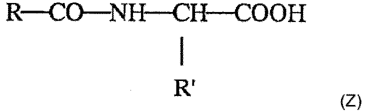 wherein R' is HOOC-CH2-CH2- or M⁺⁻OOC-CH2-CH2- wherein M⁺ is a cation; and wherein R is a linear or branched, saturated alkyl group having 6 to 30, preferably 8 to 22, more preferably 8 to 18 carbon atoms or is a linear or branched, mono- or polyunsaturated alkenyl group having 6 to 30, preferably 8 to 22, more preferably 12 to 18 carbon atoms.

In at least one embodiment, M⁺ is a cation. Preferably, M⁺ is selected from the group consisting of Li⁺, Na⁺, K⁺, Mg⁺⁺, Ca⁺⁺, Al⁺⁺⁺, NH4⁺, a monoalkylammonium ion, a dialkylammonium ion, a trialkylammonium ion and a tetraalkylammonium ion, or mixtures thereof. More preferably, M⁺ is sodium. Particularly preferably, R is C12 alkyl or C14 alkyl. Also particularly preferably, R is C16 alkyl or C18 alkyl.

A particularly preferred acyl glutamate is sodium cocoyl glutamate. Sodium cocoyl glutamate is, e.g., commercially available from Clariant (Hostapon^{®} CGN).

Acyl glutamates and in particular acyl glutamates of formula (Z) or salts thereof are beneficial because they are mild and non-irritating.

The opacifier composition of the invention comprises from 2 to 12 wt.-%, particularly preferably from 3 to 10 wt.-% of the sulfate-free anionic surfactant (b), based on the total weight of the opacifier composition. Also preferably, the opacifier composition of the invention comprises from 2 to 10 wt.-%, more preferably from 2 to 8 wt.-%, particularly preferably from 3 to 6 wt.-% of the sulfate-free anionic surfactant (b), based on the total weight of the opacifier composition.

The opacifier composition comprises
from 20 to 35 wt.-%, particularly preferably from 25 to 30 wt.-% of wax particles (a), based on the total weight of the opacifier composition; and
from 2 to 12 wt.-%, particularly preferably from 3 to 10 wt.-% of the sulfate-free anionic surfactant (b), based on the total weight of the opacifier composition.

In preferred embodiments, the opacifier composition comprises
from 20 to 35 wt.-%, particularly preferably from 25 to 30 wt.-% of wax particles (a), based on the total weight of the opacifier composition; and
from 2 to 10 wt.-%, preferably from 2 to 8 wt.-%, particularly preferably from 3 to 6 wt.-% of the sulfate-free anionic surfactant (b), based on the total weight of the opacifier composition.

The opacifier composition of the invention comprises
(a) wax particles, wherein the wax particles comprise mono- or di- C8-C20 esters of ethylene glycol, diethylene glycol or triethylene glycol, or rice bran wax, oxidized rice bran wax, rice bran wax ethyl esters, or mixtures thereof; and
(b) a sulfate-free anionic surfactant, wherein the sulfate-free anionic surfactant is selected from acyl isethionates, acyl taurates, acyl glutamates, and mixtures thereof.

In preferred embodiments, the opacifier composition of the invention comprises
(a) wax particles, wherein the wax particles comprise mono- or di- C8-C20 esters of ethylene glycol, diethylene glycol or triethylene glycol, or mixtures thereof; and
(b) a sulfate-free anionic surfactant, wherein the sulfate-free anionic surfactant is selected from acyl isethionates, acyl taurates, acyl glutamates, and mixtures thereof.

In preferred embodiments, the opacifier composition of the invention comprises
(a) wax particles, wherein the wax particles comprise mono- or di- C12-C18 esters of ethylene glycol, or mixtures thereof; and
(b) a sulfate-free anionic surfactant, wherein the sulfate-free anionic surfactant is selected from acyl isethionates, acyl taurates, acyl glutamates, and mixtures thereof.

**In** preferred embodiments, the opacifier composition of the invention comprises
(a) wax particles, wherein the wax particles comprise mono- or di- C12-C18 esters of ethylene glycol, or mixtures thereof; and
(b) a sulfate-free anionic surfactant, wherein the sulfate-free anionic surfactant is selected from acyl isethionates, acyl taurates, and mixtures thereof.

**In** preferred embodiments, the opacifier composition comprises
from 20 to 35 wt.-% of wax particles (a), based on the total weight of the opacifier composition, wherein the wax particles comprise mono- or di- C8-C20 esters of ethylene glycol, or mixtures thereof; and
from 2 to 12 wt.-% of the sulfate-free anionic surfactant (b), based on the total weight of the opacifier composition, wherein the sulfate-free anionic surfactant is selected from acyl isethionates, acyl taurates, acyl glutamates, and mixtures thereof.

**In** preferred embodiments, the opacifier composition comprises
from 25 to 30 wt.-% of wax particles (a), based on the total weight of the opacifier composition, wherein the wax particles comprise mono- or di- C8-C20 esters of ethylene glycol, or mixtures thereof; and
from 3 to 10 wt.-% of the sulfate-free anionic surfactant (b), based on the total weight of the opacifier composition, wherein the sulfate-free anionic surfactant is selected from acyl isethionates, acyl taurates, acyl glutamates, and mixtures thereof.

**In** preferred embodiments, the opacifier composition comprises
from 20 to 35 wt.-% of wax particles (a), based on the total weight of the opacifier composition, wherein the wax particles comprise mono- or di- C12-C18 esters of ethylene glycol, or mixtures thereof; and
from 2 to 12 wt.-% of the sulfate-free anionic surfactant (b), based on the total weight of the opacifier composition, wherein the sulfate-free anionic surfactant is selected from acyl isethionates, acyl taurates, and mixtures thereof.

**In** preferred embodiments, the opacifier composition comprises
from 25 to 30 wt.-% of wax particles (a), based on the total weight of the opacifier composition, wherein the wax particles comprise mono- or di- C12-C18 esters of ethylene glycol, or mixtures thereof; and
from 3 to 10 wt.-% of the sulfate-free anionic surfactant (b), based on the total weight of the opacifier composition, wherein the sulfate-free anionic surfactant is selected from acyl isethionates, acyl taurates, and mixtures thereof.

In preferred embodiments, the opacifier composition of the invention further comprises an amphoteric or zwitterionic surfactant (c).

Preferably, the amphoteric or zwitterionic surfactant comprises a betaine surfactant, more preferably comprises a C8- to C18-fatty acid alkylamidobetaine, particularly preferably comprises cocamidopropyl betaine.

Preferably, the amphoteric or zwitterionic surfactant is selected from betaine surfactants, more preferably is selected from C8- to C18-fatty acid alkylamidobetaines, particularly preferably is cocamidopropyl betaine.

The opacifier composition of the invention may comprise one or more amphoteric or zwitterionic surfactants. Preferably, the opacifier composition of the invention comprises 1 to 3 amphoteric or zwitterionic surfactants, more preferably 1 or 2 amphoteric or zwitterionic surfactants, particularly preferably 1 amphoteric or zwitterionic surfactant.

In at least one embodiment, the amphoteric or zwitterionic surfactant is selected from the group consisting of N-(C₁₂-C₁₈)-alkyl-beta-aminopropionates and N-(C₁₂-C₁₈)-alkyl-beta-iminodipropionates as alkali metal salts or mono-, di-, or trialkylammonium salts; N-acylaminoalkyl-N,N-dimethylacetobetaine, preferably N-(C₈-C₁₈)-acylaminopropyl-N,N-dimethylacetobetaine; amphosurfactants based on imidazoline (trade name: Miranol^{®}, Steinapon^{®}), preferably the sodium salt of 1-(beta-carboxymethyloxyethyl)-1-(carboxymethyl)-2-laurylimidazolinium; amine oxide, e.g. (C₁₂-C₁₈)-alkyl-dimethylamine oxide, fatty acid amidoalkyldimethylamine oxide; and mixtures thereof.

In at least one embodiment, the opacifier composition of the invention comprises a betaine surfactant. Optionally, the betaine surfactant is selected from C8- to C18-alkylbetaines. In at least one embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine, laurylbis(2-hydroxypropyl)alphacarboxyethylbetaine, and mixtures thereof. Optionally, the betaine surfactant is selected from carboxyl derivatives of imidazole, C8- to C18-alkyldimethylammonium acetates, C8- to C18-alkyldimethylcarbonylmethylammonium salts, C8- to C18-fatty acid alkylamidobetaines, and mixtures thereof.

Preferably, the betaine surfactant is selected from C8- to C18-fatty acid alkylamidobetaines. More preferably, the betaine surfactant is selected from coconut fatty acid amidopropylbetaine, N-coconut fatty acid amidoethyl-N-[2-(carboxymethoxy)ethyl]glycerol (CTFA name: cocoamphocarboxyglycinate), and mixtures thereof. Particularly preferably, the betaine surfactant is cocamidopropyl betaine. Cocamidopropyl betaine is, e.g., commercially available from Clariant (Genagen^{®} CAB 818).

A particularly preferred amphoteric or zwitterionic surfactant is cocamidopropyl betaine. Mixtures of any of the foregoing amphoteric or zwitterionic surfactants may also be suitable. Preferred mixtures are those of cocamidopropyl betaine with one or more further amphoteric or zwitterionic surfactants as described above. Another preferred amphoteric or zwitterionic surfactant is sodium cocoamphoacetate.

Preferably, the opacifier composition of the invention comprises from 0.1 to 4.5 wt.-%, more preferably from 0.2 to 3 wt.-%, particularly preferably from 0.3 to 2 wt.-% of the amphoteric or zwitterionic surfactant (c), based on the total weight of the opacifier composition.

In some embodiments, the opacifier composition of the invention comprises from 0 to 4.5 wt.-%, more preferably from 0 to 3 wt.-%, particularly preferably from 0 to 2 wt.-% of an amphoteric or zwitterionic surfactant (c), based on the total weight of the opacifier composition.

In some embodiments, the opacifier composition of the invention does not contain an amphoteric or zwitterionic surfactant.

In some embodiments, the opacifier composition of the invention does not contain a betaine surfactant.

In preferred embodiments, the opacifier composition of the invention further comprises a nonionic surfactant (d).

The opacifier composition of the invention may comprise one or more nonionic surfactants. Preferably, the opacifier composition of the invention comprises 1 to 3 nonionic surfactants, more preferably 1 or 2 nonionic surfactants, particularly preferably 1 nonionic surfactant.

Preferably, the nonionic surfactant is selected from glyceryl fatty acid esters, polyglyceryl fatty acid esters, N-methyl-N-acylglucamines, anhydro methyl glucamides, sorbitan esters, and mixtures thereof.

More preferably, the nonionic surfactant is selected from glyceryl fatty acid esters, polyglyceryl fatty acid esters, N-methyl-N-acylglucamines, and mixtures thereof. Even more preferably, the nonionic surfactant is selected from glyceryl fatty acid esters, polyglyceryl fatty acid esters, and mixtures thereof. Also even more preferably, the nonionic surfactant is selected from N-methyl-N-acylglucamines. Particularly preferably, the nonionic surfactant is selected from esters of glycerol and one or more C8-C20 fatty acids, esters of polyglycerol having 2 to 20 glyceryl units and one or more C8-C20 fatty acids, and mixtures thereof.

In at least one embodiment, the nonionic surfactant is selected from the group consisting of glyceryl fatty acid esters. Preferred glyceryl fatty acid esters are esters of glycerol and one or more C8-C20 fatty acids.

Preferably, the glyceryl fatty acid esters are mono- or diesters of glycerol and one or more C8-C20 fatty acids. Particularly preferably, the glyceryl fatty acid esters are monoesters of glycerol and one or more C8-C20 fatty acids. Also particularly preferably, the glyceryl fatty acid esters are diesters of glycerol and one or more C8-C20 fatty acids. Also particularly preferably, the glyceryl fatty acid esters are mixtures of mono- and diesters of glycerol and one or more C8-C20 fatty acids.

The fatty acids may be saturated or unsaturated. Preferred fatty acids are C12-C18 fatty acids. Preferably, the fatty acids are selected from oleic acid, capric acid, caprylic acid, lauric acid, myristic acid, palmitic acid, stearic acid, ricinoleic acid, and mixtures thereof. A particularly preferred fatty acid is oleic acid. Also preferred are fatty acid mixtures derived from coconut oil.

Examples of preferred glyceryl fatty acid esters are glyceryl oleate, glyceryl stearate, glyceryl caprate, glyceryl caprylate, glyceryl laurate, glyceryl myristate, glyceryl palmitate, glyceryl cocoate, glyceryl ricinoleate, or mixtures thereof. A particularly preferred glyceryl fatty acid ester is glyceryl oleate.

In at least one embodiment, the nonionic surfactant is selected from the group consisting of polyglyceryl fatty acid esters. Preferred polyglyceryl fatty acid esters are esters of polyglycerol having 2 to 20 glyceryl units and one or more C8-C20 fatty acids.

Preferably, the polyglyceryl fatty acid esters are mono-, di-, tri- or tetraesters, more preferably mono-, di- or triesters, even more preferably mono- or diesters of polyglycerol and one or more C8-C20 fatty acids. Particularly preferably, the polyglyceryl fatty acid esters are monoesters of polyglycerol and one or more C8-C20 fatty acids. Also particularly preferably, the polyglyceryl fatty acid esters are diesters of polyglycerol and one or more C8-C20 fatty acids. Also particularly preferably, the polyglyceryl fatty acid esters are mixtures of mono- and diesters of polyglycerol and one or more C8-C20 fatty acids.

Preferably, the polyglycerols have 2 to 4 glyceryl units, more preferably 2 or 3 glyceryl units, particularly preferably 2 glyceryl units.

In preferred embodiments, the polyglyceryl fatty acid esters are mono- or diesters of polyglycerol having 2 to 4, preferably 2 or 3, particularly preferably 2 glyceryl units and one or more C8-C20 fatty acids. In preferred embodiments, the polyglyceryl fatty acid esters are monoesters of polyglycerol having 2 to 4, preferably 2 or 3, particularly preferably 2 glyceryl units and one or more C8-C20 fatty acids. In preferred embodiments, the polyglyceryl fatty acid esters are diesters of polyglycerol having 2 to 4, preferably 2 or 3, particularly preferably 2 glyceryl units and one or more C8-C20 fatty acids. In preferred embodiments, the polyglyceryl fatty acid esters are mixtures of mono- and diesters of polyglycerol having 2 to 4, preferably 2 or 3, particularly preferably 2 glyceryl units and one or more C8-C20 fatty acids.

The fatty acids may be saturated or unsaturated. Preferred fatty acids are C12-C18 fatty acids. Preferably, the fatty acids are selected from stearic acid, capric acid, caprylic acid, lauric acid, myristic acid, palmitic acid, oleic acid, ricinoleic acid, and mixtures thereof. A particularly preferred fatty acid is stearic acid. Also preferred are fatty acid mixtures derived from coconut oil.

Examples of preferred polyglyceryl fatty acid esters are polyglyceryl-2 stearate, polyglyceryl-3 stearate, polyglyceryl-4 stearate, polyglyceryl-2 sesquistearate, polyglyceryl-2 sesquiisostearate, polyglyceryl-2 caprate, polyglyceryl-3 caprate, polyglyceryl-4 caprate, polyglyceryl-2 caprylate, polyglyceryl-3 caprylate, polyglyceryl-4 caprylate, polyglyceryl-2 laurate, polyglyceryl-3 laurate, polyglyceryl-4 laurate, polyglyceryl-2 myristate, polyglyceryl-3 myristate, polyglyceryl-4 myristate, polyglyceryl-2 palmitate, polyglyceryl-3 palmitate, polyglyceryl-4 palmitate, polyglyceryl-2 oleate, polyglyceryl-3 oleate, polyglyceryl-4 oleate, polyglyceryl-2 cocoate, polyglyceryl-3 cocoate, polyglyceryl-4 cocoate, polyglyceryl-2 ricinoleate, polyglyceryl-3 ricinoleate, polyglyceryl-4 ricinoleate, or mixtures thereof. A particularly preferred polyglyceryl fatty acid ester is polyglyceryl-2 stearate. Polyglyceryl-2 stearate is, e.g., commercially available from Clariant (Plantasens^{®} Emulsifier DGDS). Polyglyceryl-2 sesquiisostearate is, e.g. commercially available from Clariant (Plantasens^{®} Emulsifier DGI).

In at least one embodiment, the nonionic surfactant is selected from the group consisting of N-methyl-N-acylglucamines, preferably N-methyl-N-acylglucamines of formula (II): 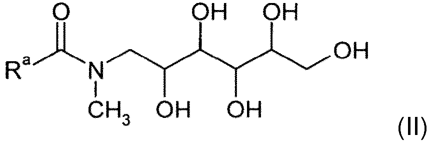 wherein R^{a} is selected from saturated or unsaturated hydrocarbon chains having 5 to 23 carbon atoms. Preferably, R^{a} in formula (II) is selected from saturated or unsaturated hydrocarbon chains having 7 to 17 carbon atoms. In preferred embodiments, R^{a} in formula (II) is selected from saturated hydrocarbon chains having 7 to 17 carbon atoms. In preferred embodiments, R^{a} in formula (II) is selected from unsaturated hydrocarbon chains having 7 to 17 carbon atoms.

Also preferably, the R^{a}-C=O residue in formula (II) is derived from caprylic acid, capric acid, lauric acid, myristic acid, palmitic acid, stearic acid, oleic acid, linoleic acid, linolenic acid, or mixtures thereof. Also preferably, the R²-C=O residue in formula (II) is derived from coconut oil. Also preferably, the R²-C=O residue in formula (II) is derived from 9-decenoic acid, 9-dodecenoic acid, or mixtures thereof.

Particularly preferred N-methyl-N-acylglucamines of formula (II) are capryloyl/caproyl methyl glucamide, lauroyl/myristoyl methyl glucamide, cocoyl methyl glucamide, oleyl methyl glucamide, or mixtures thereof. Such N-methyl-N-acylglucamines are commercially available from Clariant (GlucoTain^{®} Clear, GlucoTain^{®} Plus, GlucoTain^{®} Flex, GlucoTain^{®} Care, GlucoTain^{®} Sense).

Also particularly preferred N-methyl-N-acylglucamines of formula (II) are N-9-decenoyl-N-methylglucamine, N-9-dodecenoyl-N-methylglucamine, or mixtures thereof.

In at least one embodiment, the nonionic surfactant is selected from the group consisting of anhydro methyl glucamides, preferably anhydro methyl glucamides of formula (I), 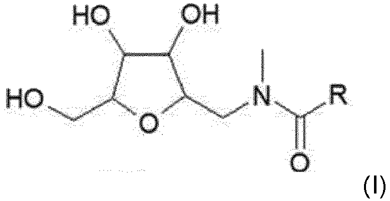 wherein R is selected from saturated or unsaturated hydrocarbon chains having 5 to 23 carbon atoms. Preferably, R in formula (I) is selected from saturated or unsaturated hydrocarbon chains having 7 to 17 carbon atoms. More preferably, R in formula (I) is selected from saturated or unsaturated hydrocarbon chains having 7 to 13 carbon atoms. Even more preferably, R in formula (I) is -(CH₂)₆CH₃, -(CH₂)₈CH₃, - (CH₂)₁₀CH₃, -(CH₂)₁₂CH₃, or mixtures thereof. Also even more preferably, the R-C=O residue in formula (I) is derived from coconut oil. Also even more preferably, the R-C=O residue in formula (I) is derived from 9-decenoic acid, 9-dodecenoic acid, or mixtures thereof. Particularly preferably, R in formula (I) is -(CH₂)₆CH₃, -(CH₂)₈CH₃, or mixtures thereof. Capryloyl/caproyl anhydro methyl glucamide is commercially available from Clariant (Velsan^{®} Flex).

In at least one embodiment, the nonionic surfactant is selected from sorbitan esters. Preferred sorbitan esters are selected from sorbitan caprylate, sorbitan stearate, sorbitan isostearate, sorbitan olivate, sorbitan oleate, sorbitan sesquioleate, sorbitan laurate, and sorbitan palmitate. A particularly preferred sorbitan ester is sorbitan caprylate. Sorbitan caprylate is commercially available from Clariant (Velsan^{®} SC).

In at least one embodiment, the nonionic surfactant is selected from the group consisting of condensation products of aliphatic primary or secondary linear or branched alcohols or phenols with alkylene oxides, typically ethylene oxide, and generally having from 6 to 30 ethylene oxide groups. Alkyl ethoxylates are particularly preferred. Most preferred are alky ethoxylates having the formula

R-(OCH₂CH₂)ₙOH,

where R is an alkyl chain of C12 to C15, and n is 5 to 9. Other suitable nonionic surfactants are mono- or di-alkyl alkanolamides. Examples include coco mono- or diethanolamide and coco mono-isopropanolamide.

In at least one embodiment, the nonionic surfactant is selected from the group consisting of alkyl polyglycosides. Typical alkyl polyglycosides comprise an alkyl group connected (optionally via a bridging group) to a block of one or more glycosyl groups. Preferred alkyl polyglycosides are defined by the following formula:

RO-(G)ₙ

wherein R is a branched or straight chain alkyl group which may be saturated or unsaturated and G is a saccharide group. R may represent a mean alkyl chain length of from C5 to C20. Preferably R represents a mean alkyl chain length of from C9 to C12. G may be selected from C5 or C6 monosaccharide residues, and is preferably a glucoside. G may be selected from the group consisting of glucose, xylose, lactose, fructose, mannose, and derivatives thereof. Preferably G is glucose. The degree of polymerisation, n, may have a value of from 1 to 10 or more. Most preferably, the value of n is from 1.3 to 1.5.

In at least one embodiment, the nonionic surfactant is selected from the group consisting of ethoxylated fatty alcohols, fatty acids, fatty acid glycerides or alkylphenols, in particular addition products of from 2 to 30 mol of ethylene oxide and/or 1 to 5 mol of propylene oxide onto C8- to C22-fatty alcohols, onto C12- to C22-fatty acids or onto alkyl phenols having 8 to 15 carbon atoms in the alkyl group, C12- to C22-fatty acid mono- or diesters of addition products of from 1 to 30 mol of ethylene oxide onto glycerol, addition products of from 5 to 60 mol of ethylene oxide onto castor oil or onto hydrogenated castor oil, fatty acid sugar esters, in particular esters of sucrose and one or two C8- to C22-fatty acids, e.g. Sucrose Cocoate, Sucrose Dilaurate, Sucrose Distearate, Sucrose Laurate, Sucrose Myristate, Sucrose Oleate, Sucrose Palmitate, Sucrose Ricinoleate or Sucrose Stearate, esters of sorbitan and one, two or three C8- to C22-fatty acids and a degree of ethoxylation of from 4 to 20, alkyl glucosides, alkyl oligoglucosides or alkyl polyglucosides having C8 to C22-alkyl groups, e.g. decylglucoside or laurylglucoside, and mixtures thereof.

**In** at least one embodiment, the nonionic surfactant is selected from the group consisting of fatty alcohol ethoxylates (alkylpolyethylene glycols), alkylphenol polyethylene glycols, alkylmercaptan polyethylene glycols, fatty amine ethoxylates (alkylaminopolyethylene glycols), fatty acid ethoxylates (acylpolyethylene glycols), polypropylene glycol ethoxylates, fatty acid alkylol amides (fatty acid amide polyethylene glycols), N-alkoxypoly-hydroxy-fatty acid amides, sucrose esters, sorbitol esters, polyglycol ethers, and mixtures thereof.

Preferably, the opacifier composition of the invention comprises from 0.1 to 5 wt.-%, more preferably from 0.3 to 3.5 wt.-%, particularly preferably from 0.5 to 2.5 wt.-% of the nonionic surfactant (d), based on the total weight of the opacifier composition.

**In** some embodiments, the opacifier composition of the invention comprises from 0 to 5 wt.-%, more preferably from 0 to 3.5 wt.-%, particularly preferably from 0 to 2.5 wt.-% of the nonionic surfactant (d), based on the total weight of the opacifier composition.

**In** some embodiments, the opacifier composition of the invention does not contain a nonionic surfactant.

**In** preferred embodiments, the opacifier composition of the invention further comprises a diluent (e). Advantageously, the diluent is cosmetically acceptable.

Preferred diluents are water, alcohols, or mixtures thereof. More preferred diluents are water, glycerin, ethanol, propanol, isopropanol, ethylene glycol, propylene glycol, or mixtures thereof. A particularly preferred diluent is water. Water is particularly useful for environmental and economic reasons.

Preferably, the opacifier composition of the invention comprises from 30 to 75 wt.-%, more preferably from 45 to 75 wt.-%, particularly preferably from 50 to 70 wt.-% of the diluent (e), based on the total weight of the opacifier composition.

Optionally, the opacifier composition of the invention comprises further additives (f). Examples of further additives are fatty acids or salts thereof.

**In** some embodiments, the opacifier composition of the invention further comprises a fatty acid or a salt thereof. Preferred fatty acids are stearic acid, capric acid, caprylic acid, lauric acid, myristic acid, palmitic acid, oleic acid, ricinoleic acid, or mixtures thereof. A particularly preferred fatty acid is stearic acid. Also preferred are fatty acid mixtures derived from coconut oil. Preferred salts are sodium or potassium salts. More preferred salts are sodium salts.

Preferably, the opacifier composition of the invention comprises from 0 to 10 wt.-%, more preferably from 0 to 5 wt.-%, particularly preferably from 0 to 3 wt.-% of further additives (f), based on the total weight of the opacifier composition.

**In** preferred embodiments, the opacifier composition of the invention comprises
(a) wax particles;
(b) a sulfate-free anionic surfactant; and
(c) an amphoteric or zwitterionic surfactant.

**In** preferred embodiments, the opacifier composition of the invention comprises
(a) wax particles;
(b) a sulfate-free anionic surfactant; and
(d) a nonionic surfactant.

**In** preferred embodiments, the opacifier composition of the invention comprises
(a) wax particles;
(b) a sulfate-free anionic surfactant;
(c) an amphoteric or zwitterionic surfactant; and
(d) a nonionic surfactant.

**In** preferred embodiments, the opacifier composition of the invention comprises
(a) from 20 to 35 wt.-% of wax particles, based on the total weight of the opacifier composition;
(b) from 2 to 12 wt.-% of a sulfate-free anionic surfactant, based on the total weight of the opacifier composition;
(d) from 0.1 to 5 wt.-% of a nonionic surfactant, based on the total weight of the opacifier composition.

**In** more preferred embodiments, the opacifier composition of the invention comprises
(a) from 20 to 35 wt.-% of wax particles, based on the total weight of the opacifier composition;
(b) from 2 to 12 wt.-% of a sulfate-free anionic surfactant, based on the total weight of the opacifier composition;
(d) from 0.3 to 3.5 wt.-% of a nonionic surfactant, based on the total weight of the opacifier composition.

**In** even more preferred embodiments, the opacifier composition of the invention comprises
(a) from 25 to 30 wt.-% of wax particles, based on the total weight of the opacifier composition;
(b) from 3 to 10 wt.-% of a sulfate-free anionic surfactant, based on the total weight of the opacifier composition;
(d) from 0.5 to 2.5 wt.-% of a nonionic surfactant, based on the total weight of the opacifier composition.

**In** preferred embodiments, the opacifier composition of the invention comprises
(a) from 20 to 35 wt.-% of wax particles, based on the total weight of the opacifier composition;
(b) from 2 to 12 wt.-% of a sulfate-free anionic surfactant, based on the total weight of the opacifier composition;
(c) from 0.1 to 4.5 wt.-% of an amphoteric or zwitterionic surfactant, based on the total weight of the opacifier composition; and
(d) from 0.1 to 5 wt.-% of a nonionic surfactant, based on the total weight of the opacifier composition.

**In** more preferred embodiments, the opacifier composition of the invention comprises
(a) from 20 to 35 wt.-% of wax particles, based on the total weight of the opacifier composition;
(b) from 2 to 12 wt.-% of a sulfate-free anionic surfactant, based on the total weight of the opacifier composition;
(c) from 0.2 to 3 wt.-% of an amphoteric or zwitterionic surfactant, based on the total weight of the opacifier composition; and
(d) from 0.3 to 3.5 wt.-% of a nonionic surfactant, based on the total weight of the opacifier composition.

**In** even more preferred embodiments, the opacifier composition of the invention comprises
(a) from 25 to 30 wt.-% of wax particles, based on the total weight of the opacifier composition;
(b) from 3 to 10 wt.-% of a sulfate-free anionic surfactant, based on the total weight of the opacifier composition;
(c) from 0.3 to 2 wt.-% of an amphoteric or zwitterionic surfactant, based on the total weight of the opacifier composition; and
(d) from 0.5 to 2.5 wt.-% of a nonionic surfactant, based on the total weight of the opacifier composition.

**In** preferred embodiments, the opacifier composition of the invention comprises
(a) wax particles, wherein the wax particles comprise mono- or di- C8-C20 esters of ethylene glycol, diethylene glycol or triethylene glycol, or rice bran wax, oxidized rice bran wax, rice bran wax ethyl esters, or mixtures thereof;
(b) a sulfate-free anionic surfactant, wherein the sulfate-free anionic surfactant is selected from acyl isethionates, acyl taurates, acyl glutamates, and mixtures thereof; and
(d) a nonionic surfactant, wherein the nonionic surfactant is selected from glyceryl fatty acid esters, polyglyceryl fatty acid esters, N-methyl-N-acylglucamines, and mixtures thereof.

**In** preferred embodiments, the opacifier composition of the invention comprises
(a) wax particles, wherein the wax particles comprise mono- or di- C12-C18 esters of ethylene glycol, or mixtures thereof;
(b) a sulfate-free anionic surfactant, wherein the sulfate-free anionic surfactant is selected from acyl isethionates, acyl taurates, and mixtures thereof; and
(d) a nonionic surfactant, wherein the nonionic surfactant is selected from glyceryl fatty acid esters, polyglyceryl fatty acid esters, and mixtures thereof.

**In** preferred embodiments, the opacifier composition of the invention comprises
(a) wax particles, wherein the wax particles comprise mono- or di- C8-C20 esters of ethylene glycol, diethylene glycol or triethylene glycol, or rice bran wax, oxidized rice bran wax, rice bran wax ethyl esters, or mixtures thereof;
(b) a sulfate-free anionic surfactant, wherein the sulfate-free anionic surfactant is selected from acyl isethionates, acyl taurates, acyl glutamates, and mixtures thereof;
(c) an amphoteric or zwitterionic surfactant, wherein the amphoteric or zwitterionic surfactant comprises a betaine surfactant; and
(d) a nonionic surfactant, wherein the nonionic surfactant is selected from glyceryl fatty acid esters, polyglyceryl fatty acid esters, N-methyl-N-acylglucamines, and mixtures thereof.

**In** preferred embodiments, the opacifier composition of the invention comprises
(a) wax particles, wherein the wax particles comprise mono- or di- C12-C18 esters of ethylene glycol, or mixtures thereof;
(b) a sulfate-free anionic surfactant, wherein the sulfate-free anionic surfactant is selected from acyl isethionates, acyl taurates, and mixtures thereof;
(c) an amphoteric or zwitterionic surfactant, wherein the amphoteric or zwitterionic surfactant comprises a C8- to C18-fatty acid alkylamidobetaine, for example cocamidopropyl betaine; and
(d) a nonionic surfactant, wherein the nonionic surfactant is selected from glyceryl fatty acid esters, polyglyceryl fatty acid esters, and mixtures thereof.

In preferred embodiments, the opacifier composition of the invention does not contain a sulfate-containing surfactant. The term "sulfate-containing surfactant" as used herein refers to a surfactant which bears at least one sulfate group or group -OSO₃H.

In preferred embodiments, the opacifier composition of the invention is sulfate-free. This means that, in preferred embodiments, the opacifier composition of the invention does not contain a compound which bears a sulfate group or a group -OSO₃H.

In preferred embodiments, the opacifier composition of the invention does not contain a sulfate-containing surfactant selected from alkyl sulfates, alkyl ether sulfates and alkylamide sulfates. In preferred embodiments, the opacifier composition of the invention does not contain a sulfate-containing surfactant selected from sodium lauryl sulfate (SLS), sodium laureth sulfate (SLES), ammonium lauryl sulfate and ammonium laureth sulfate.

In preferred embodiments, the opacifier composition of the invention is ethylene oxide-free. This means that, in preferred embodiments, the opacifier composition of the invention does not contain a compound which bears one or more ethyleneoxy (-CH₂CH₂O-) groups.

In preferred embodiments, the opacifier composition of the invention is propylene oxide-free. This means that, in preferred embodiments, the opacifier composition of the invention does not contain a compound which bears one or more propyleneoxy (-CH(CH₃)CH₂O- or -CH₂CH(CH₃)O-) groups.

In preferred embodiments, the opacifier composition of the invention is ethylene oxide-free and propylene oxide-free.

In preferred embodiments, the opacifier composition of the invention is ethylene oxide-free, propylene oxide-free and sulfate-free.

In preferred embodiments, the opacifier composition of the invention has a pH value of from 3 to 7, preferably of from 4 to 6, particularly preferably of from 4 to 5. In preferred embodiments, the opacifier composition of the invention has a pH value of from 2 to 5, preferably of from 2 to 4, particularly preferably of from 2.5 to 3.5. In particularly preferred embodiments, the opacifier composition of the invention has a pH value of 3. The pH value can, for example, be adjusted using citric acid, hydrochloric acid or sodium hydroxide.

In preferred embodiments, the wax particles (a) have a median particle size (D50) of from 0.5 to 18 µm, preferably of from 1 to 15 µm, more preferably of from 1 to 10 µm, even more preferably of from 1 to 8 µm, particularly preferably of from 2 to 5 µm.

The D50 value indicates that 50 vol.% of the particles in the composition have a particle size which is equal to or smaller than the respective specified value. D50 is the median value of the particle size distribution and is determined via static light scattering using a Horiba LA 960 particle size analyzer and using 1.456 + 0.1i and 1.333 as refractive indexes of the dispersed and continuous phases, respectively.

In preferred embodiments, the opacifier composition of the invention has a viscosity of from 1 to 50 Pas, preferably of from 2 to 40 Pas, more preferably of from 3 to 30 Pas, particularly preferably of from 3 to 20 Pas. The viscosity as used herein is measured at a shear rate of 0.1 s⁻¹ at 25°C using a TA Instruments AR200 rheometer.

In preferred embodiments, the opacifier composition of the invention has a renewable carbon index (RCI) of at least 90%, preferably at least 95%, particularly preferably at least 96%. The renewable carbon index (RCI) as used herein is determined according to ISO 16128. Water is excluded from the calculation. The reference number of ISO 16128 as referred to herein is ISO 16128-1:2016(E).

The opacifier composition of the invention can be prepared by methods known in the art. For example, the opacifier composition of the invention can be prepared by mixing its ingredients.

The invention further relates to a method of preparing the opacifier composition of the invention, comprising the step of mixing its ingredients.

The invention also relates to the use of the opacifier composition of the invention as an opacifier in a cosmetic composition. Preferred opacifier compositions are described herein. Preferred cosmetic compositions are described herein.

The invention also relates to a cosmetic composition comprising the opacifier composition of the invention. Preferred opacifier compositions are described herein.

Preferred cosmetic compositions are hair care compositions or skin care compositions. Particularly preferred compositions are hair care compositions. Also particularly preferred compositions are skin care compositions.

Preferred cosmetic compositions are hair cleansing compositions or skin cleansing compositions. Particularly preferred compositions are hair cleansing compositions. Also particularly preferred compositions are skin cleansing compositions.

The cosmetic composition of the invention can be in the form of a rinse-off product or leave-on product. It can be formulated in a wide variety of product forms, including creams, gels, emulsions, mousses or sprays. Preferably, the cosmetic composition of the invention is in the form of a rinse-off product.

Particularly preferably, the cosmetic composition of the invention is a hair cleansing and/or skin cleansing composition in the form of a rinse-off product.

For example, the cosmetic composition of the invention may be selected from the group consisting of shampoo, cream rinse, body wash, shower gel, hand soap, bubble bath, facial cleanser, cleansing mask, make-up remover, soaps and cleansing foams.

In preferred embodiments, the cosmetic composition is a shampoo, a shower gel or a hand soap. In particularly preferred embodiments, the cosmetic composition is a shampoo. In particularly preferred embodiments, the cosmetic composition is a shower gel. In particularly preferred embodiments, the cosmetic composition is a hand soap.

Preferably, the cosmetic composition of the invention comprises from 0.5 to 5 wt.-%, more preferably from 1 to 3 wt.-%, particularly preferably from 1 to 2 wt.-% of the opacifier composition, based on the total weight of the cosmetic composition.

The cosmetic composition of the invention preferably comprises one or more further components (F), which can be in an amount of at least 0.01% by weight, preferably at least 0.05% by weight, more preferably at least 0.1% by weight, even more preferably at least 0.5% by weight, such as 0.5 to 50% by weight, preferably 0.5 to 35% by weight, more preferably 0.5 to 25% by weight, even more preferably 0.5 to 20% by weight of the cosmetic composition.

Preferably, the component (F) is selected from the group consisting of acidity regulators, colorants, conditioning agents, emulsifiers, film formers, fragrances, glossers, humectants, lubricants, moisturizers, pigments, preservatives, skin penetration enhancers, stabilizers, surfactants, thickeners, and viscosity modifiers.

More preferably, the component (F) is selected from the group consisting of acidity regulators, glossers, lubricants, and surfactants.

Suitable lubricants are, for example, fatty alcohol components having 6 to 18 carbon atoms.

The surfactants may, for example, be selected from non-polymeric, cationic quaternary ammonium compounds, in particular cetrimonium chloride (CTAC).

Suitable classical cationic conditioning agents include cationic quaternary ammonium salts. In at least one embodiment, the component (F) is a cationic quaternary ammonium salt. Examples of such quaternary ammonium salts include benzyl triethyl ammonium chloride, cetyl trimethylammonium chloride (cetrimonium chloride, CTAC), behentrimonium chloride (BTAC) or cetylpyridinium chloride.

As cationic components, a variety of cationic polymers are suitable, including quaternized cellulose ethers, copolymers of vinylpyrrolidone, acrylic polymers, including homopolymers or copolymers of dimethyldiallylammonium chloride or acrylamide. Also suitable are various types of homo- or copolymers derived from acrylic or methacrylic acid, acrylamide, methylacrylamide, diacetone-acrylamide.

In at least one embodiment, the component (F) is a glosser. Typical glossers are silicones. Suitable as silicones are volatile or nonvolatile non-ionic silicone fluids, silicone resins, and silicone semisolids or solids. Volatile silicones are linear or cyclic silicones having a measureable vapor pressure, which is defined as a vapor pressure of at least 2 mm of mercury at 20°C. Also suitable are water insoluble nonvolatile silicone fluids including polyalkyl siloxanes, polyaryl siloxanes, polyalkylaryl siloxanes, polyether siloxane copolymers, amine-functional silicones, or mixtures thereof.

The cosmetic composition of the invention may contain from 0.05 to 5%, preferably 0.5 to 5% by weight of at least one oil component. Typical oils are organic oils, which often are esters. The oil component may comprise glyceryl esters of fatty acids, or triglycerides, coconut oil, almond oil, apricot kernel oil, avocado oil, babassu oil, evening primrose oil, camelina sativa seed oil, grape seed oil, macadamia ternifolia seed oil, corn oil, meadowfoam seed oil, mink oil, olive oil, palm kernel oil, safflower oil, sesame oil, soybean oil, sunflower oil, wheat germ oil, and camellia reticulata seed oil.

The cosmetic composition of the invention may contain from 0.05 to 5%, preferably 0.5 to 5% by weight of at least one emulsifier. Preferred emulsifiers are, for example, sorbitan esters.

The cosmetic composition of the invention can contain from 0.1 to 10% by weight, preferably from 0.2 to 5% by weight, more preferably from 0.2 to 3% by weight, also more preferably from 0.5 to 5% by weight of at least one rheology modifying agent, in particular a gelling and thickening agent. Examples are cellulosic thickeners, for example, hydroxyethylcellulose, hydroxypropylcellulose, and carboxymethylcellulose, guar gum, such as hydroxypropylguar, gums of microbial origin, such as xanthan gum and scleroglucan gum, and synthetic thickeners, such as crosslinked homo- or copolymers of acrylic acid and/or of acrylamidopropanesulfonic acid. Other rheology modifying agents include fatty acid amides such as coconut diethanolamide and monoethanolamide, and oxyethylenated monoethanolamide of carboxylic acid alkyl ether.

Rheology modifying agents are also known as structuring materials. Common structuring materials include polymeric materials known as "carbomers", including, for example the cross-linked polyacrylic acid polymers available from Lubrizol Corporation under the trademark Carbopol^{®}. Another class of (meth)acylic acid polymers are alkali-swellable emulsion (ASE) polymers. ASE polymers include, for example, Aculyn^{®} 38 copolymer from Dow. Carbomers and ASE polymers belong to a class of materials known as hydrodynamic thickeners. These hydrodynamic thickeners include acid groups in their polymeric structure that, when deprotonated, form anionic charges that repel each other, causing the polymer chains to expand and entangle. Expansion and chain entanglement can give rise to thickening and suspending effects provided by the deprotonated polymers. The properties of these hydrodynamic thickeners are impacted by their molecular weight, acid group content, degree of cross-linking, and extent of swelling. These thickeners are also known as "space filling" or "volume excluding", and tend to increase both viscosity and yield point as the concentration thereof is increased. In use, hydrodynamic polymers commonly give rise to compositions that exhibit shear thinning or non-Newtonian behavior. Another class of (meth)acrylic acid based rheology modifiers are hydrophobically modified alkali swellable (HASE) polymers. Like ASE polymers, the HASE polymers include acid groups, the deprotonation of which gives rise to polymer swelling. Additionally, the HASE polymers include hydrophobic side groups, chains or blocks that give rise to associative interactions with each other, as well as with other hydrophobic species present in the compositions in which they are employed, for example, hydrophobic groups of surfactants, fatty acids, other thickening agents, and the like. Association creates hydrophobic regions distributed throughout the polymer chain network. This can also help to enhance the properties of the materials as solubilizing agents. Aculyn^{®} 22 and Aculyn^{®} 28 copolymers from Dow and Aqua SF 1^{®} copolymer from Lubrizol Corporation are among the commonly used HASE materials. U.S. Patent 4,529,773 (Witiak et al.) reports alkali-soluble emulsion polymers activated by neutralization to a pH above 6.5, and subsequently acidified in the presence of a surfactant. These are described as useful thickeners in acidic compositions. The polymers are formed from the copolymerization of a monomer system that includes: (1) methacrylic or acrylic acid, (2) methacrylic or acrylic acid ester of a C8-C30 alkyl or, as therein more particularly described, a hydrocarbyl monoether of polyethylene glycol, (3) a C1-C4 alkyl acrylate or methacrylate, and, optionally, (4) a small amount of a polyethylenically unsaturated monomer.

The cosmetic composition of the invention can also comprise as component (F) a fatty compound. The fatty compound may be included in the cosmetic composition at a level of from 0.1 to 20 % by weight, preferably from 1.0 to 10% by weight. The fatty compound is selected from the group consisting of fatty alcohols (e.g. cetyl alcohol, stearyl alcohol or cetearyl alcohol), fatty acids, fatty alcohol derivatives, fatty acid derivatives, or mixtures thereof.

It is understood that the components disclosed can in some instances fall into more than one classification, e.g., some fatty alcohol derivatives can also be classified as fatty acid derivatives. However, a given classification, is not intended to be a limitation on that particular compound but is done so for convenience of classification and nomenclature. Non-limiting examples are found in International Cosmetic Ingredient Dictionary and Handbook, Fourteenth Edition (2014), and CTFA Cosmetic Ingredient Handbook, Second Edition, 1992. Preferably, fatty alcohols have 14 to 30 or 16 to 22 carbon atoms. These fatty alcohols are saturated and can be linear or branched. Examples of fatty alcohols are cetyl alcohol, stearyl alcohol, behenyl alcohol, and mixtures thereof. Preferred fatty acids have from 10 to 30 or from 12 to 22 carbon atoms. These fatty acids can be saturated and can be linear or branched. Also included herein are salts of these fatty acids. Examples of fatty acids are lauric acid, palmitic acid, stearic acid, behenic acid, sebacic acid, or mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, or mixtures thereof. Examples of fatty alcohol derivatives and fatty acid derivatives include methyl stearyl ether, polyoxyethylene ethers of behenyl alcohol, ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, or mixtures thereof.

The cosmetic composition of the invention may comprise an aqueous carrier. The level and species of the aqueous carrier are selected according to the compatibility with other components and other desired characteristic of the cosmetic composition. The aqueous carrier may, for example, be water or water solutions of lower alkyl alcohols or polyhydric alcohols. The lower alkyl alcohols may, for example, be monohydric alcohols having 1 to 6 carbons, often ethanol and/or isopropanol. The polyhydric alcohols may, for example, be propylene glycol, hexylene glycol, glycerin, and/or propane diol. Preferably, the aqueous carrier is substantially water. Deionized water is preferably used. Water from natural sources, including minerals can also be used, depending on the desired characteristic of the composition. Generally, the cosmetic composition of the invention can comprise up to 80 %, often even up to 95 % by weight of water.

The cosmetic composition of the invention may also include as a further component (F) other components being suitable for rendering the compositions more cosmetically or aesthetically acceptable or to provide them with additional usage benefits. Such other components can generally be used individually at levels of from 0.001 % to 5 % by weight. A wide variety of further components (F) can be formulated into the cosmetic composition of the invention. These include conditioning agents, such as panthenol, panthenyl ethyl ether, proteins, hydrolysed proteins (preferably of vegetable or animal origin, for example hydrolysed collagen or hydrolysed keratin), nutrients; antioxidants, such as vitamin E; emollients, such as PPG-3 myristyl ether, trimethyl pentanol hydroxyethyl ether; hair-fixative polymers, such as amphoteric fixative polymers, cationic fixative polymers, anionic fixative polymers, non-ionic fixative polymers, silicone grafted copolymers; preservatives, such as benzyl alcohol, methyl paraben, propyl paraben, imidazolidinyl urea; pH adjusting agents, such as citric acid, sodium citrate, succinic acid, phosphoric acid, sodium hydroxide, sodium carbonate; salts, in general, such as potassium acetate or sodium chloride; coloring agents; hair oxidizing (bleaching) agents, such as hydrogen peroxide, perborate or persulfate salts; hair reducing agents such as thioglycolates; perfumes; and sequestering agents, such as disodium ethylene-diamine tetraacetate; ultraviolet and infrared screening and absorbing agents, such as octyl salicylate; anti-dandruff agents, such as zinc pyrithione, piroctone olamine or salicylic acid.

In at least one embodiment, the cosmetic composition of the invention comprises an anti-fungal substance. In at least one embodiment, the anti-fungal substance is selected from the group consisting of ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, zinc pyrithione, piroctone olamine (Octopirox^{®}), and mixtures thereof. In at least one embodiment, the cosmetic composition of the invention comprises a total amount of anti-fungal substance of from 0.1 wt.-% to 1 wt.-%. In at least one embodiment, the cosmetic composition of the invention comprises piroctone olamine. In at least one embodiment, the cosmetic composition of the invention comprises a pyridinethione anti-dandruff particulate. For example, 1-hydroxy-2-pyridinethione salts are highly preferred particulate anti-dandruff agents. The concentration of pyridinethione anti-dandruff particulate may range from 0.1% to 4% by weight of the composition, preferably from 0.1% to 3%, more preferably from 0.3% to 2%. Preferred pyridinethione salts include those formed from heavy metals such as zinc, tin, cadmium, magnesium, aluminum or zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form. Salts formed from other cations, such as sodium, may also be suitable. Pyridinethione anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982.

Preferably, salt is present at levels from 0.1 to 1 wt.-% of the total cosmetic composition to adjust the product viscosity. Preferably, NaOH is present at levels from 0.1 to 1 wt.-% of the total cosmetic composition to adjust the pH of the formulation.

The cosmetic composition of the invention may contain as a further component (F) a polysorbate for adjusting rheology, for example, polysorbate-20, polysorbate-21, polysorbate-40, polysorbate-60, or mixtures thereof. The polysorbate can be contained in the cosmetic composition in amounts up to 5% (e.g. 0.1 to 5%) by weight.

The cosmetic composition of the invention can also contain as a further component (F) a polypropylene glycol. Preferred polypropylene glycols are those having a weight average molecular weight of from 200 to 100000 g/mol. The polypropylene glycol may be either water-soluble, water-insoluble, or may have a limited solubility in water, depending upon the degree of polymerization and whether other moieties are attached thereto. The desired solubility of the polypropylene glycol in water will depend in large part upon the form of the composition (e.g., leave-on composition, rinse-off composition). The polypropylene glycol can be included in the cosmetic composition of the invention at a level of up to 10% by weight.

For example, in a rinse-off composition, it is preferred that the polypropylene glycol has a solubility in water at 25°C of less than about 1 g/100 g water, more preferably a solubility in water of less than about 0.5 g/100 g water, and even more preferably a solubility in water of less than about 0.1 g/100 g water. The polypropylene glycol can be included in the cosmetic composition of the invention at a level of up to 10% by weight.

The cosmetic composition of the invention can also contain, as a further component (F), low melting point oil selected from the group consisting of hydrocarbons having from 10 to 40 carbon atoms; unsaturated fatty alcohols having from 10 to 30 carbon atoms such as oleyl alcohol; unsaturated fatty acids having from about 10 to about 30 carbon atoms; fatty acid derivatives; fatty alcohol derivatives; ester oils such as pentaerythritol ester oils, trimethylol ester oils, citrate ester oils, or glyceryl ester oils; poly [alpha]-olefin oils; and mixtures thereof. Preferred low melting point oils are selected from the group consisting of ester oils such as pentaerythritol ester oils, trimethylol ester oils, citrate ester oils, or glyceryl ester oils; poly [alpha]-olefin oils; and mixtures thereof. Particularly useful pentaerythritol ester oils and trimethylol ester oils are pentaerythritol tetraisostearate, pentaerythritol tetraoleate, trimethylolpropane triisostearate, trimethylolpropane trioleate, or mixtures thereof. Particularly useful glyceryl esters are triisostearin, triolein or trilinolein.

The cosmetic composition of the invention can also contain, as a further component (F), a cationic polymer. Cationic polymers may be present in the cosmetic composition of the invention for further enhancing deposition performance.

Suitable cationic polymers may be homopolymers which are cationically substituted or may be formed from two or more types of monomers. The weight average (Mw) molecular weight of the polymers will generally be between 100 000 and 2 million g/mol. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain non-cationic spacer monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol. The cationic amines can be primary, secondary or tertiary amines, depending upon the particular species and the pH of the composition. In general, secondary and tertiary amines, especially tertiary, are preferred. Amine substituted vinyl monomers and amines can be polymerized in the amine form and then converted to ammonium by quaternization. The cationic polymers can comprise mixtures of monomer units derived from amine- and/or quaternary ammonium-substituted monomer and/or compatible spacer monomers.

Suitable cationic polymers include, for example cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; mineral acid salts of amino-alkyl esters of homo- and co-polymers of unsaturated carboxylic acids having from 3 to 5 carbon atoms, (as described in US4009256A1 from NAT STARCH CHEM CORP); cationic polyacrylamides (as described in WO95/22311A1 Unilever PLC).

Other cationic polymers that can be used include cationic polysaccharide polymers, such as cationic cellulose derivatives, cationic starch derivatives, and cationic guar gum derivatives.

Cationic polysaccharide polymers suitable for use in the cosmetic composition of the invention include monomers of the formula: A-O-[R-N⁺(R1)(R2)(R3)X⁻], wherein: A is an anhydroglucose residual group, such as a starch or cellulose anhydroglucose residual. R is an alkylene, oxyalkylene, polyoxyalkylene, or hydroxyalkylene group, or combination thereof. R1, R2 and R3 independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R1, R2 and R3) is preferably about 20 or less, and X⁻ is an anionic counterion. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the trade name Polymer LM-200. Other suitable cationic polysaccharide polymers include quaternary nitrogen-containing cellulose ethers (e.g. as described in US3962418 from L'Oréal), and copolymers of etherified cellulose and starch (e.g. as described in US3958581 from L'Oréal).

A particularly suitable type of cationic polysaccharide polymer that can be used is a cationic guar gum derivative, such as guar hydroxypropyltrimethylammonium chloride (commercially available from Solvay in their JAGUAR trade named series). Examples of such materials are JAGUAR C13S, JAGUAR C14, JAGUAR C15, JAGUAR C17, JAGUAR C16, JAGUAR CHT and JAGUAR C162.

Mixtures of any of the above cationic polymers may be used. Cationic polymer may be present in the cosmetic composition of the invention at levels of from 0.01 to 5 wt.-%, preferably from 0.05 to 1 wt.-%, more preferably from 0.08 to 0.5 wt.-% by total weight of cationic polymer based on the total weight of the cosmetic composition.

In at least one embodiment, the cationic polymers have a number average molecular weight of at least about 5000 g/mol, typically from 10000 g/mol to 10 million g/mol and are selected from the group consisting of copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol, maleic anhydride, propylene glycol, and ethylene glycol. Preferred cationic polymers are cationic celluloses, cationic starches, and cationic guar gums. Commercially available cationic guar polymers are e.g. Jaguar^{®} from Solvay.

In at least one embodiment, the cosmetic composition of the invention comprises a surfactant system. In at least one embodiment, the surfactant system comprises a surfactant selected from the group consisting of anionic surfactants, cationic surfactants, non-ionic surfactants, zwitterionic surfactants and/or amphoteric surfactants. In at least one embodiment, the cosmetic composition of the invention comprises a total amount of surfactant of from 0.01 wt.-% to 70 wt.-%, preferably from 0.1 wt.-% to 40%, more preferably from 1 wt.-% to 30%, particularly preferably from 2 wt.-% to 20 wt.-%.

In at least one embodiment, the cosmetic composition of the invention comprises an anionic surfactant (in addition to the anionic surfactant present in the opacifier composition). Anionic surfactants that may be present in the cosmetic composition are those that are described herein as anionic surfactants that may be present in the opacifier composition.

Other anionic surfactants that may be present in the cosmetic composition are the following: fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, alpha-sulfo fatty acid esters, alkylbenzenesulfonates, alkylphenol glycol ether sulfonates, sulfosuccinates, sulfosuccinic monoesters and diesters, alkyl monoglyceride sulfates and sulfonates, alkylglyceride ether sulfonates, sulforicinoleates, and mixtures thereof. The anionic surfactants (and their mixtures) can be used in the form of their water-soluble or water-dispersible salts, examples being the sodium, potassium, magnesium, ammonium, mono-, di- and triethanolammonium, and analogous alkylammonium salts. Examples of anionic surfactants for use in the cosmetic composition include sodium lauryl sulfate, sodium laureth sulfate, sodium tridecyl sulfate, sodium trideceth sulfate, sodium myristyl sulfate, sodium myreth sulfate, and mixtures thereof. Examples of anionic surfactants for use in the cosmetic composition include ammonium lauryl sulfosuccinate, sodium lauryl sulfate, sodium lauryl ether sulfate, sodium lauryl ether sulfosuccinate, ammonium lauryl sulfate, ammonium lauryl ether sulfate, sodium dodecyl benzene sulfonate, triethanolamine dodecylbenzene sulfonate, and mixtures thereof.

The cosmetic composition of the invention may, for example, comprise from 0.5 wt.-% to 45 wt.-%, preferably from 1 wt.-% to 30 wt.-%, more preferably from 2 wt.-% to 25 wt.-%, more preferably from 5 wt.-% to 20 wt.-%, more preferably from 12 wt.-% to 18 wt.-% anionic surfactant.

In at least one embodiment, the cosmetic composition of the invention comprises a nonionic surfactant. Nonionic surfactants that may be present in the cosmetic composition are those that are described herein as nonionic surfactants that may be present in the opacifier composition. The cosmetic composition of the invention may, for example, comprise from 0.5 wt.-% to 20 wt.-%, preferably from 1 wt.-% to 10 wt.-%, more preferably from 2 wt.-% to 5 wt.-% non-ionic surfactant.

In at least one embodiment, the cosmetic composition of the invention comprises an amphoteric or zwitterionic surfactant. Amphoteric or zwitterionic surfactants that may be present in the cosmetic composition are those that are described herein as amphoteric or zwitterionic surfactants that may be present in the opacifier composition. The cosmetic composition of the invention may, for example, comprise from 0.5 wt.-% to 20 wt.-%, preferably from 1 wt.-% to 10 wt.-%, more preferably from 2 wt.-% to 5 wt.-% amphoteric or zwitterionic surfactant.

In at least one embodiment, the cosmetic composition of the invention comprises a surfactant system. In at least one embodiment, the surfactant system comprises at least one surfactant selected from the group consisting of sodium lauryl sulfate, sodium laureth sulfate, cocamidopropyl betaine, sodium cocoylglutamate, lauroamphoacetate, and mixtures thereof. **In** at least one embodiment, the surfactant system comprises sodium laureth sulfate, sodium lauryl sulfate, and optionally cocamidopropyl betaine. In at least one embodiment, the surfactant system comprises sodium laureth sulfate, potassium cocoylglutamate, and cocamidopropyl betaine.

In at least one embodiment, the cosmetic composition of the invention comprises as a further component a silicone compound. The cosmetic composition can comprise up to 5% (e.g. 0.1 to 5%) by weight of a silicone compound. Suitable silicone compounds include polyalkyl or polyaryl siloxanes. The preferred silicone compounds are polydimethylsiloxane, polydiethylsiloxane, and polymethylphenylsiloxane, e.g. available from Wacker (Germany) or Dow Corning, such as Xiameter PMX DC 200. Silicone compounds can be available as silicone oils or emulsions. The silicone compounds may further be incorporated in the present composition in the form of an emulsion, wherein the emulsion is pre-made and added to the formulation, or made during the formulation process by mechanical mixing with or without the aid of an additional surfactant selected from anionic surfactants, non-ionic surfactants, cationic surfactants, and mixtures thereof.

In at least one embodiment, the cosmetic composition of the invention comprises silicone conditioning agents. Preferably, these are emulsified droplets of a silicone conditioning agent. These are for enhancing conditioning performance.

Suitable silicones include polydiorganosiloxanes, in particular polydimethylsiloxanes, which have the CTFA designation dimethicone. Also suitable for use in the cosmetic composition of the invention are polydimethyl siloxanes having hydroxyl end groups, which have the CTFA designation dimethiconol. Also suitable for use in the cosmetic composition of the invention are silicone gums having a slight degree of cross-linking, as are described for example in WO 96/31188. The viscosity of the emulsified silicone itself (not the emulsion or the final composition) is typically at least 10,000 cSt at 25°C. The viscosity of the silicone itself is preferably at least 60,000 cSt, most preferably at least 500,000 cSt, ideally at least 1,000,000 cSt. Preferably, the viscosity does not exceed 1x10⁹ cSt for ease of formulation. Emulsified silicones for use in the cosmetic composition of the invention will typically have an average silicone droplet size in the composition of less than 30, preferably less than 20, more preferably less than 10 micron, ideally from 0.01 to 1 micron. Silicone emulsions having an average silicone droplet size of less than 0.15 micron are generally termed microemulsions.

Silicone particle size may be measured by means of a laser light scattering technique, for example using a 2600D Particle Sizer from Malvern Instruments. Examples of suitable pre-formed emulsions include Xiameter MEM 1785 and microemulsion DC2-1865 available from Dow Corning. These are emulsions / microemulsions of dimethiconol. Cross-linked silicone gums are also available in a pre-emulsified form, which is advantageous for ease of formulation. A further preferred class of silicones for inclusion in the cosmetic composition of the invention are amino functional silicones. By "amino functional silicone" is meant a silicone containing at least one primary, secondary or tertiary amine group, or a quaternary ammonium group. Examples of suitable amino functional silicones include polysiloxanes having the CTFA designation "amodimethicone". Specific examples of amino functional silicones suitable for use in the cosmetic composition of the invention are the aminosilicone oils DC2-8220, DC2-8166 and DC2-8566 (all ex Dow Corning). Suitable quaternary silicone polymers are described in EP-A-0 530 974. A preferred quaternary silicone polymer is K3474, ex Goldschmidt.

Also suitable are emulsions of amino functional silicone oils with non-ionic and/or cationic surfactants. Pre-formed emulsions of amino functional silicones are also available from suppliers of silicone oils such as Dow Corning and General Electric. Specific examples include DC939 Cationic Emulsion and the non-ionic emulsions DC2-7224, DC2- 8467, DC2-8177 and DC2-8154 (all ex Dow Corning).

Combination of amino and non-amino functional silicones may also be used.

The total amount of silicone is preferably from 0.01 wt.-% to 10 wt.-% of the total composition, more preferably from 0.1 wt.-% to 5 wt.-%, most preferably from 0.5 wt.-% to 3 wt.-%.

In at least one embodiment, the cosmetic composition of the invention comprises a preservative or preservative system. Examples of suitable preservatives include benzyl alcohol, piroctone olamine, phenoxyethanol, parabens, pentanediol, benzoic acid/sodium benzoate, sorbic acid/potassium sorbate, or combinations thereof. Examples of suitable preservation boosting ingredients include anisic acid, lactic acid, sorbitan caprylate, ethylhexylglycerin, caprylyl glycol, octanediol, or combinations thereof. In at least one embodiment, the cosmetic composition of the invention comprises from 0.01 to 5 wt.-%, particularly preferably from 0.05 to 1 wt.-% of at least one preservative. Suitable preservatives are the substances listed in the International Cosmetic Ingredient Dictionary and Handbook, 9th Edition with the function "preservatives".

In at least one embodiment, the cosmetic composition of the invention comprises a preservative selected from the group consisting of cetyltrimethyl ammonium chloride, cetylpyridinium chloride, benzethonium chloride, diisobutylethoxyethyldimethyl benzylammonium chloride, sodium N-lauryl sarcosinate, sodium N-palmethylsarcosinate, lauroylsarcosine, N-myristoylglycine, potassium-N-laurylsarcosine, trimethylammonium chloride, sodium aluminium chlorohydroxylactate, triethylcitrate, tricetylmethylammonium chloride, 2,4,4'-trichloro-2'-hydroxydiphenylether (Triclosan), phenoxyethanol, 1,5-pentanediol, 1,6-hexanediol, 3,4,4'-trichlorocarbanilide (Triclocarban), diaminoalkylamide, L-lysine hexadecylamide, heavy metal citrate salts, salicylate, piroctose, zinc salts, pyrithione and its heavy metal salts, zinc pyrithione, zinc phenol sulfate, farnesol, ketoconazol, oxiconazol, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, selenium disulfide, methylchloroisothiazolinone, methylisothiazolinone, methyldibromo glutaronitrile, piroctone olamine (Octopirox^{®}), AgCl, chloroxylenol, sodium salts of diethylhexylsulfosuccinate, sodium benzoate, phenoxyethanol, benzyl alcohol, phenoxyisopropanol, paraben, such as butyl-, ethyl-, methyl- and propylparaben, and their salts, pentanediol, 1,2-octanediol, ethylhexylglycerin, benzyl alcohol, sorbic acid, benzoic acid, lactic acid, imidazolidinyl urea, diazolidinyl urea, dimethylol dimethyl hydantoin (DMDMH), sodium salts of hydroxymethyl glycinate, hydroxyethylglycine of sorbic acid, and combinations thereof. In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin, and combinations thereof. In at least one embodiment, the cosmetic composition of the invention is substantially free of parabens.

The cosmetic composition of the invention may also comprise a dispersed, nonvolatile, water-insoluble oily conditioning agent. By "insoluble" is meant that the material is not soluble in water (distilled or equivalent) at a concentration of 0.1 % (w/w), at 25°C.

Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C2-C6 alkenyl monomers. Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms and include esters with hydrocarbyl chains derived from fatty acids or alcohols. Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used. Particularly preferred fatty esters are mono-, di- and triglycerides, more specifically the mono-, di-, and tri-esters of glycerol and long chain carboxylic acids such as C8-C22 carboxylic acids. Preferred materials include cocoa butter, palm stearin, sunflower oil, soybean oil and coconut oil.

The oily or fatty material may be present at a level of from 0.05 to 10 wt.-%, preferably from 0.2 to 5 wt.-%, more preferably from 0.5 to 3 wt.-%, based on the total weight of the cosmetic composition.

In at least one embodiment, the cosmetic composition of the invention is sulfate-free. In at least one embodiment, the cosmetic composition of the invention is silicone-free. In at least one embodiment, the cosmetic composition of the invention is sulfate-free and silicone-free.

The cosmetic composition of the invention can be prepared by methods known in the art. For example, the cosmetic composition of the invention can be prepared by mixing its ingredients.

The invention also relates to a method of treating hair and/or skin, comprising:
a) applying the cosmetic composition of the invention onto hair and/or skin and then
b) removing the cosmetic composition from the hair and/or scalp.

The invention also relates to the use of the opacifier composition of the invention as an opacifier in a liquid dishwashing or laundry detergent. The invention also relates to a liquid dishwashing or laundry detergent comprising the opacifier composition of the invention. Preferred opacifier compositions are described herein. The liquid dishwashing or laundry detergent can be for manual use or for use in an automatic dish or laundry machine. The liquid dishwashing or laundry detergent encompasses unit dose type detergents, aqueous detergents, concentrated detergents, heavy duty detergents, and light duty detergents. Preferably, the liquid dishwashing or laundry detergent of the invention comprises from 0.5 to 5 wt.-%, more preferably from 1 to 3 wt.-%, particularly preferably from 1 to 2 wt.-% of the opacifier composition, based on the total weight of the liquid dishwashing or laundry detergent.

The liquid dishwashing or laundry detergent can comprise one or more further components. Preferably, the liquid dishwashing or laundry detergent comprises one or more surfactants, in particular one or more surfactants selected from the group consisting of anionic surfactants, cationic surfactants, non-ionic surfactants, zwitterionic surfactants and/or amphoteric surfactants. Surfactants that may be present in the liquid dishwashing or laundry detergent are those that are described herein as surfactants that may be present in the cosmetic composition.

The invention is further illustrated by the following examples, without being limited thereby.

### Examples

### Examples 1 to 10

### Opacifier compositions (ingredients are given in wt%)

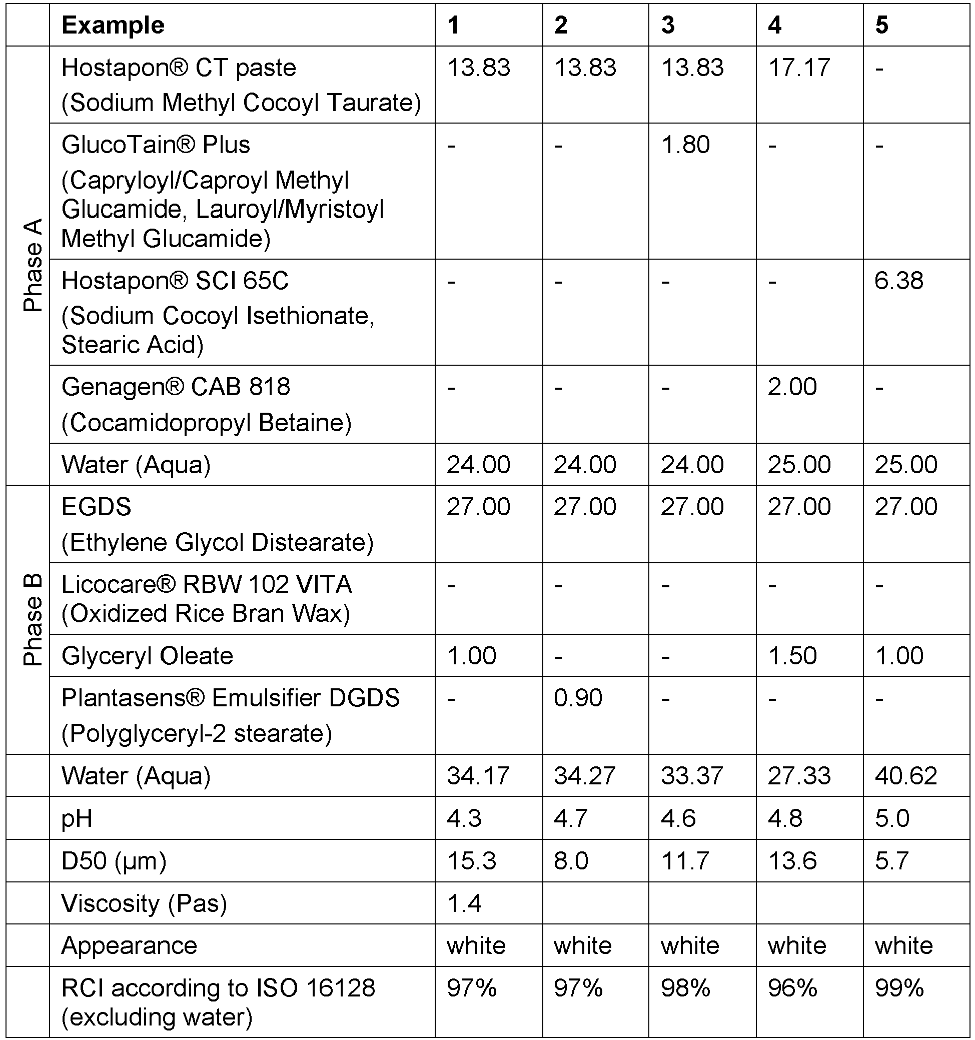

### Opacifier compositions (ingredients are given in wt%)

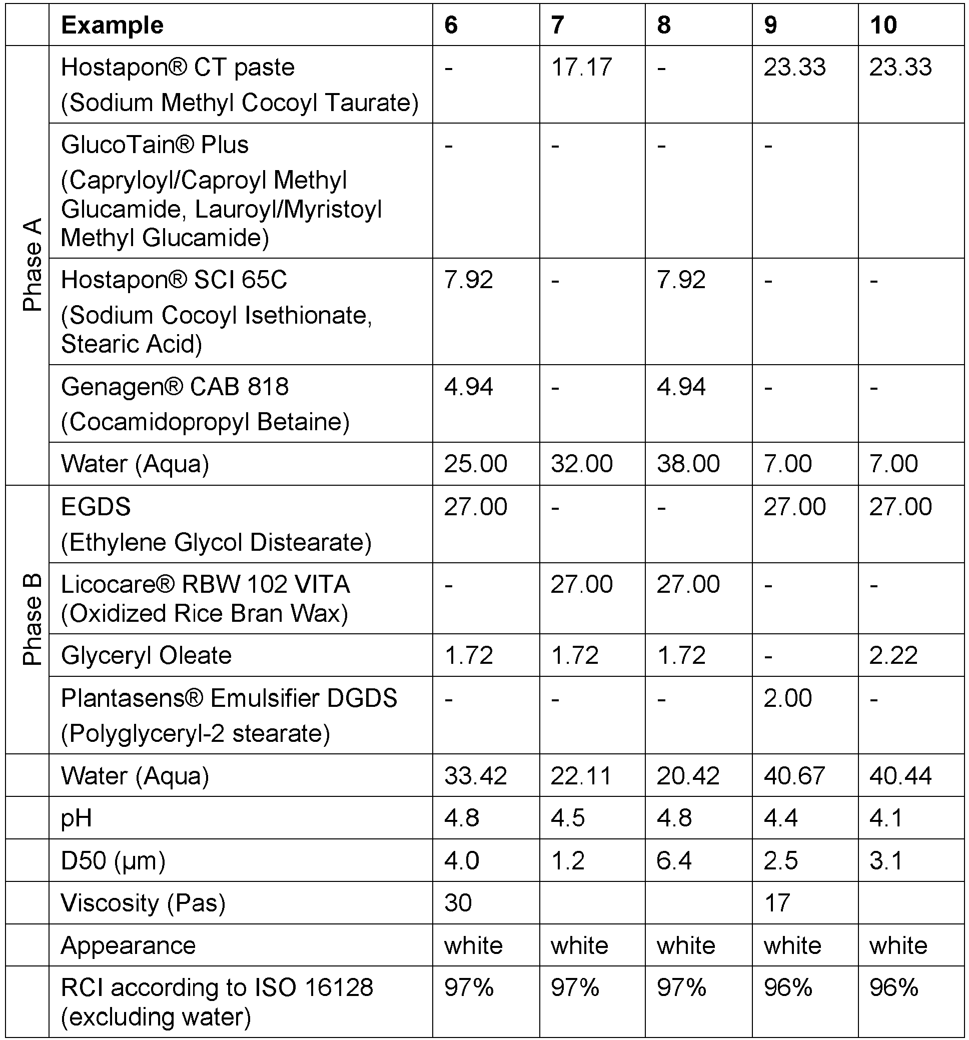

### Opacifier compositions (ingredients are given in wt%)

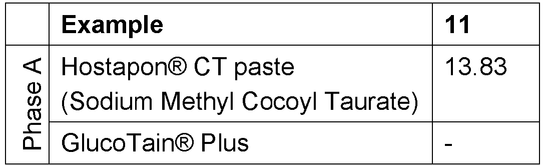

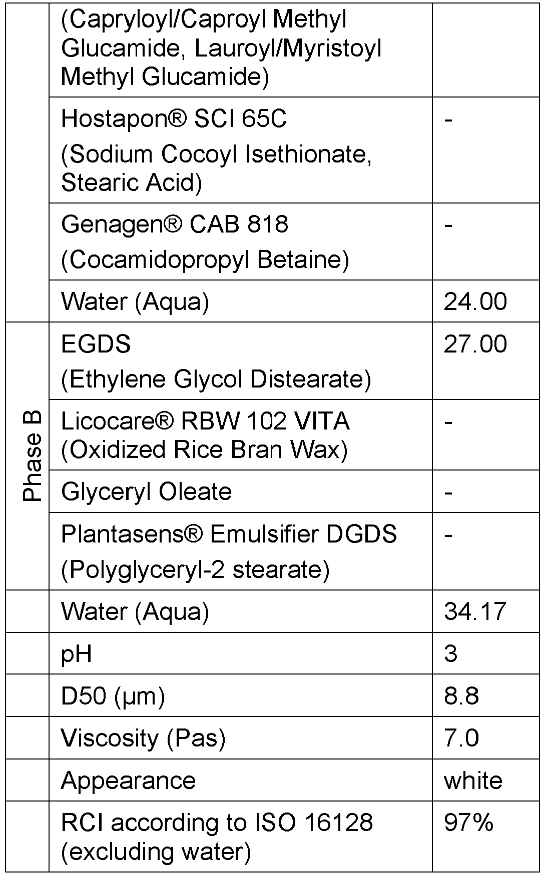

The opacifier compositions according to Examples 1 to 11 have all a milky turbid appearance and do not show any pearlescent effect.

The following ingredients were used:
- Hostapon^{®} CT paste (Sodium Methyl Cocoyl Taurate), 30% active substance
- GlucoTain^{®} Plus (Capryloyl/Caproyl Methyl Glucamide, Lauroyl/Myristoyl Methyl Glucamide), 50% active substance
- Hostapon^{®} SCI 65C (Sodium Cocoyl Isethionate, Stearic Acid), 65% active substance
- Genagen^{®} CAB 818 (Cocamidopropyl Betaine), 30% active substance
- EGDS (Ethylene Glycol Distearate), 100% active substance
- Licocare^{®} RBW 102 VITA (Oxidized Rice Bran Wax), 100% active substance
- Glyceryl Oleate, 90% active substance
- Plantasens^{®} Emulsifier DGDS (Polyglyceryl-2 stearate), 100% active substance

The opacifier compositions were prepared as follows:
An aqueous phase A was prepared at 25°C by mixing emulsifiers and water and then warmed to 80 - 90 °C. Phase B was also warmed to 80 - 90°C and then added under continuous stirring to phase A. Warm water (80 - 90°C) was added to the resulting mixture to yield a milky-turbid emulsion, which was allowed to cool down to room temperature under stirring. The pH of the emulsion was adjusted to 2.5 - 5 by addition of citric acid solution 50wt%.

D50 is the median value of the particle size distribution and was determined via static light scattering using a Horiba LA 960 particle size analyzer and using 1.456 + 0.1i and 1.333 as refractive indexes of the dispersed and continuous phases, respectively.

The viscosity of the emulsion was determined by measuring a shear rate-controlled flow curve from 0.01 to 100 s⁻¹ at 25°C with a TA Instruments AR200 rheometer. The indicated value of the viscosity was measured at a shear rate of 0.1 s⁻¹.

### Examples 12a-d

### Cosmetic compositions (ingredients are given in wt%)

| **Example** | **12a** | **12b** | **12c** | **12d** |
|---|---|---|---|---|
| Composition | Liquid soap | Body wash | Shampoo | Shampoo |
| Opacifier composition according to Example 5 | 1.5 | 2.0 | 1.0 | 3.0 |
| SLES | 10 | - | - | 9 |
| Cocamidopropyl Betaine | 2 | 4.5 | - | 2 |
| Cocamide MEA | - | - | - | 1 |
| Sodium Cocoyl Glutamate | - | 2 | 2 | - |
| Sodium Cocoyl Glycinate | - | 2 | 2 | - |
| Sodium Cocoyl Isethionate | - | 2 | - | - |
| Cocoyl Methyl Glucamide | - | 3 | - | - |
| Cocoyl Glucoside | - | - | 5 | - |
| Lauryl Glucoside | - | - | 5 | - |
| Acrylates Copolymer | - | - | 2 | - |
| Glycerin | - | - | 1 | 2 |
| Panthenol | - | - | 0.2 | 0.2 |
| Polyquaternium 7 (PQ-7) | - | - | - | 0.6 |
| Guar Hydroxypropyltrimonium Chloride | - | - | - | 0.3 |
| Hydrolyzed Protein | - | - | - | 0.1 |
| N-fatty acyl-N-methyl Cyclic glucamide | 1.5 | 2 | 1.5 | 2 |
| NaCl | 1.5 | 1.0 | 0.3 | 1.5 |
| Water | QSP | QSP | QSP | QSP |
| Total | 100 | 100 | 100 | 100 |

### Examples 12e-i

### Cosmetic compositions (ingredients are given in wt%)

| **Example** | **12e** | **12f** | **12g** | **12h** | **12i** |
|---|---|---|---|---|---|
| Composition | Soap formulation | Body wash | Shampoo | Wash cream | Body wash |
| Opacifier composition according to Example 9 | 1.0 | 1.5 | 2.5 | 2.0 | 1.5 |
| Sodium Lauryl Sulfate | - | 10 | - | - | - |
| Ammonium Lauryl Sulfate | - | - | 12 | - | - |
| Sodium Lauryl Ether Sulfate (SLES) | 15 | 2 | - | - | - |
| Cocamidopropyl Betaine | 7 | 2 | 3 | - | 2 |
| Cocamide MEA | - | 1 | - | - | 2 |
| Lauric Acid | 0.5 | - | - | - | 10 |
| Myristic Acid | 1.5 | - | - | - | 10 |
| Stearic Acid | 0.5 | - | - | 10 | - |
| Palmitic Acid | - | - | - | - | 10 |
| Potassium Tallowate | - | - | - | - | - |
| Sodium Palm Kernelate | - | - | - | - | - |
| Sodium Cocoate | - | - | - | - | - |
| Lauryl Glucoside | - | - | 4 | - | - |
| Sodium Cocoyl Isethionate | - | - | - | 14 | - |
| Glycerin | - | - | - | - | 5 |
| Cetearyl Alcohol | 1.5 | - | - | 2 | - |
| Dimethicone | - | - | - | 0.1 | - |
| Polyquaternium 7 (PQ-7) | - | - | 0.2 | - | - |
| Guar Hydroxypropyltrimonium Chloride | - | 0.3 | - | - | - |
| Hydrolyzed Protein | - | - | 0.1 | - | - |
| N-Fatty Acyl-N-Methyl Cyclic Glucamide | 1.5 | 1.0 | 1.5 | 1 | 0.8 |
| Sodium Chloride | 1.5 | 0.5 | 0.3 | - | 2.5 |
| Water | QSP | QSP | QSP | QSP | QSP |
| Total | 100 | 100 | 100 | 100 | 100 |

### Example 13

### Liquid soap (ingredients are given in wt%)

| Water | Ad 100 % |
|---|---|
| Opacifier composition according to Example 10 | 1.5 % |
| Glycerin | 3 % |
| 1,2-Propanediol | 2 % |
| Aristoflex BLV (Clariant) | 0.4 % |
| Ammonium Acrylodimethyltaurate/Beheneth-25 Methacrylate Crosspolymer | |
| Genapol^{®} LRO liquid (Clariant) | 15 % |
| Sodium Laureth Sulfate | |
| Genagen^{®} CAB 818 (Clariant) | 4 % |
| Cocamidopropyl Betaine | |
| GlucoTain^{®} Clear (Clariant) | 2 % |
| Capryloyl/Caproyl Methyl Glucamide | |
| Nipaguard^{™} DMDMH (Clariant) | 0.4 % |
| DMDM Hydantoin | |
| Fragrance | 0.2 % |
| Sodium Cloride | 0.5 % |

### Example 14

### Effect shower gel (ingredients are given in wt%)

| Water | Ad 100 % |
|---|---|
| Opacifier composition according to Example 2 | 1.0 % |
| Genapol^{®} LRO liquid (Clariant) | 30 % |
| Sodium Laureth Sulfate | |
| Genagen^{®} CAB 818 (Clariant) | 6 % |
| Cocamidopropyl Betaine | |
| Hostapon^{®} KCG (Clariant) | 5 % |
| Sodium Cocoyl Glutamate | |
| Aristoflex TAC (Clariant) | 1.4 % |
| Ammonium Acrylodimethyltaurate/Carboxethyl Acrylate Crosspolymer | |
| Nipaguard^{®} DMDMH (Clariant) | 0.5 % |
| DMDM Hydantoin | |

### Example 15

### Shampoo compositions comprising the following ingredients:

| **Example** | **15a** | **15b** | **15c** | **15d** |
|---|---|---|---|---|
| Ingredient | wt% | wt% | wt% | wt% |
| Aqua | To 100 | To 100 | To 100 | To 100 |
| Opacifier composition according to Example 1 | 2.5 | - | - | - |
| Opacifier composition according to Example 2 | - | 1.0 | - | - |
| Opacifier composition according to Example 3 | - | - | 3.0 | - |
| Opacifier composition according to Example 4 | - | - | - | 1.5 |
| Sodium Laureth Sulfate | 14 | 14 | 14 | 14 |
| Coco-Betaine | 4 | 4 | 4 | 4 |
| Dimethicone | 2 | 2 | 2 | 2 |
| Sodium Chloride | 0.5 | 0.5 | 0.5 | 0.5 |
| Olive Fruit Oil | 0.4 | 0.4 | 0.4 | 0.4 |
| PPG-5-Ceteth-20 | 0.3 | 0.3 | 0.3 | 0.3 |
| Polyquaternium-10 | 0.25 | 0.25 | 0.25 | 0.25 |
| Carbomer | 0.2 | 0.2 | 0.2 | 0.2 |
| Genamin^{®} CTAC (Clariant) | 0.15 | 0.15 | 0.15 | 0.15 |
| Cetrimonium Chloride | | | | |
| Citric Acid | As needed, pH 4.5 - 6.5 | | | |
| Sodium Hydroxide | As needed, pH 4.5 - 6.5 | | | |
| Colorant | As needed | | | |

### Example 16

### Shampoo compositions comprising the following ingredients:

| **Example** | **16a** | **16b** | **16c** | **16d** |
|---|---|---|---|---|
| Ingredient | wt% | wt% | wt% | wt% |
| Aqua | To 100 | To 100 | To 100 | To 100 |
| Opacifier composition according to Example 5 | 2.5 | - | - | - |
| Opacifier composition according to Example 6 | - | 1.5 | - | - |
| Opacifier composition according to Example 7 | - | - | 1.5 | - |
| Opacifier composition according to Example 8 | - | - | - | 1.0 |
| Ammonium Lauryl Sulfate | 16 | 16 | 16 | 16 |
| Cocoamidopropyl Betaine | 5 | 5 | 5 | 5 |
| Sodium Chloride | 2.5 | 2.5 | 2.5 | 2.5 |
| Niacinamide | 2 | 2 | 2 | 2 |
| Genamin^{®} KDMP (Clariant) Behentrimonium Chloride | 0.2 | 0.2 | 0.2 | 0.2 |
| Propylene Glycol | 0.1 | 0.1 | 0.1 | 0.1 |
| Citric Acid | As needed, pH 4.5 - 6.5 | | | |
| Sodium Hydroxide | As needed, pH 4.5 - 6.5 | | | |
| Colorant | As needed | | | |

### Example 17

### Shampoo compositions comprising the following ingredients:

| **Example** | **17a** | **17b** | **17c** | **17d** |
|---|---|---|---|---|
| Ingredient | wt% | wt% | wt% | wt% |
| Aqua | To 100 | To 100 | To 100 | To 100 |
| Opacifier composition according to Example 9 | 1.0 | - | - | - |
| Opacifier composition according to Example 10 | - | 1.5 | - | - |
| Opacifier composition according to Example 1 | - | - | 2.2 | - |
| Opacifier composition according to Example 2 | - | - | - | 1.8 |
| Sodium Laureth Sulfate | 16 | 16 | 16 | 16 |
| Coco-Betaine | 2.5 | 2.5 | 2.5 | 2.5 |
| Glycerin | 2 | 2 | 2 | 2 |
| Sodium Chloride | 1.5 | 1.5 | 1.5 | 1.5 |
| Dimethicone | 1.5 | 1.5 | 1.5 | 1.5 |
| Piroctone Olamine | 1.5 | 1.5 | 1.5 | 1.5 |
| Cocamide MIPA | 0.5 | 0.5 | 0.5 | 0.5 |
| PPG-5-Ceteth-20 | 0.2 | 0.2 | 0.2 | 0.2 |
| Fumaric Acid | 0.15 | 0.15 | 0.15 | 0.15 |
| Carbomer | 0.15 | 0.15 | 0.15 | 0.15 |
| Citric Acid | As needed, pH 4.5 - 6.5 | | | |
| Sodium Hydroxide | As needed, pH 4.5 - 6.5 | | | |
| Colorant | As needed | | | |

### Example 18

### Shampoo compositions comprising the following ingredients:

| **Example** | **18a** | **18b** | **18c** | **18d** |
|---|---|---|---|---|
| Ingredient | wt% | wt% | wt% | wt% |
| Aqua | To 100 | To 100 | To 100 | To 100 |
| Opacifier composition according to Example 3 | 1.0 | - | - | - |
| Opacifier composition according to Example 4 | - | 1.5 | - | - |
| Opacifier composition according to Example 5 | - | - | 2.0 | - |
| Opacifier composition according to Example 6 | - | - | - | 3.0 |
| Sodium Laureth Sulfate | 16 | 16 | 16 | 16 |
| Coco-Betaine | 4 | 4 | 4 | 4 |
| Cocamide MIPA | 1.5 | 1.5 | 1.5 | 1.5 |
| Sodium Chloride | 1.5 | 1.5 | 1.5 | 1.5 |
| Zinc Pyrithione | 1.0 | 1.0 | 1.0 | 1.0 |
| Sodium Cocoate | 0.8 | 0.8 | 0.8 | 0.8 |
| Cocamide MIPA | 0.5 | 0.5 | 0.5 | 0.5 |
| Olea Europaea Oil (Olive) Fruit Oil | 0.3 | 0.3 | 0.3 | 0.3 |
| PEG-60 Hydrogenated Castor Oil | 0.3 | 0.3 | 0.3 | 0.3 |
| Polyquaternium-7 | 0.2 | 0.2 | 0.2 | 0.2 |
| Amodimethicone | 0.15 | 0.15 | 0.15 | 0.15 |
| Citric Acid | As needed, pH 4.5 - 6.5 | | | |
| Sodium Hydroxide | As needed, pH 4.5 - 6.5 | | | |
| Colorant | As needed | | | |

### Example 19

### Shampoo compositions comprising the following ingredients:

| **Example** | **19a** | **19b** | **19c** | **19d** |
|---|---|---|---|---|
| Ingredient | wt% | wt% | wt% | wt% |
| Aqua | To 100 | To 100 | To 100 | To 100 |
| Opacifier composition according to Example 7 | 1.0 | - | - | - |
| Opacifier composition according to Example 8 | - | 2.0 | - | - |
| Opacifier composition according to Example 9 | - | - | 2.5 | - |
| Opacifier composition according to Example 10 | - | - | - | 3.0 |
| Sodium Cocoyl Isethionate | 4 | 4 | 4 | 4 |
| Sodium Lauryl Sulfoacetate | 3 | 3 | 3 | 3 |
| Disodium Laureth Sulfosuccinate | 2.5 | 2.5 | 2.5 | 2.5 |
| Cocamidopropyl Betaine | 2.5 | 2.5 | 2.5 | 2.5 |
| Sodium Lauroyl Sarcosinate | 2.5 | 2.5 | 2.5 | 2.5 |
| Glycereth-26 | 1.8 | 1.8 | 1.8 | 1.8 |
| Decyl Glucoside | 1.5 | 1.5 | 1.5 | 1.5 |
| Divinyldimethicone/Dimethicone Copolymer | 1.2 | 1.2 | 1.2 | 1.2 |
| Sodium Chloride | 1.1 | 1.1 | 1.1 | 1.1 |
| Amodimethicone | 1 | 1 | 1 | 1 |
| Polyquaternium-7 | 1 | 1 | 1 | 1 |
| Sodium Isethionate | 0.5 | 0.5 | 0.5 | 0.5 |
| Propylene Glycol | 0.4 | 0.4 | 0.4 | 0.4 |
| Carbomer | 0.4 | 0.4 | 0.4 | 0.4 |
| Glycerin | 0.3 | 0.3 | 0.3 | 0.3 |
| Citric Acid | As needed, pH 4.5 - 6.5 | | | |
| Sodium Hydroxide | As needed, pH 4.5 - 6.5 | | | |
| Colorant | As needed | | | |

### Example 20

### Shampoo compositions comprising the following ingredients:

| **Example** | **20a** | **20b** | **20c** | **20d** |
|---|---|---|---|---|
| Ingredient | wt% | wt% | wt% | wt% |
| Aqua | To 100 | To 100 | To 100 | To 100 |
| Opacifier composition according to Example 7 | 1.0 | - | - | - |
| Opacifier composition according to Example 8 | - | 2.0 | - | - |
| Opacifier composition according to Example 9 | - | - | 3.0 | - |
| Opacifier composition according to Example 10 | - | - | - | 1.5 |
| Sodium Laureth Sulfate | 37 | 37 | 37 | 37 |
| Lauroyl Methyl Glucamide | 5.7 | 5.7 | 5.7 | 5.7 |
| Sodium Cocoyl Isethionate | 3.3 | 3.3 | 3.3 | 3.3 |
| Hydroxypropyl Guar (and) Hydroxypropyl Guar Hydroxypropyltrimonium Chloride | 0.2 | 0.2 | 0.2 | 0.2 |
| Acrylates/C10-30 Alkyl Acrylate Crosspolymer | 0.4 | 0.4 | 0.4 | 0.4 |
| Trideceth-9 PG Amodimethicone (and) Trideceth-12 | 0.5 | 0.5 | 0.5 | 0.5 |
| Benzophenone-4 | 0.2 | 0.2 | 0.2 | 0.2 |
| Sodium Benzoate | 0.45 | 0.45 | 0.45 | 0.45 |
| Fragrance | 0.2 | 0.2 | 0.2 | 0.2 |
| Sodium Chloride | 2.0 | 2.0 | 2.0 | 2.0 |
| Citric Acid | As needed, pH 4.5 - 6.5 | | | |
| Sodium Hydroxide | As needed, pH 4.5 - 6.5 | | | |
| Colorant | As needed | | | |

### Example 21

### Body wash compositions comprising the following ingredients:

| **Example** | **21a** | **21b** | **21c** | **21d** |
|---|---|---|---|---|
| Ingredient | wt% | wt% | wt% | wt% |
| Aqua | To 100 | To 100 | To 100 | To 100 |
| Opacifier composition according to Example 2 | 1.5 | - | - | - |
| Opacifier composition according to Example 5 | - | 2.0 | - | - |
| Opacifier composition according to Example 6 | - | - | 2.0 | - |
| Opacifier composition according to Example 9 | - | - | - | 1.5 |
| Hostapur^{®} OS liq. (Clariant) Sodium C14-16 Olefin Sulfonate | 21 | 21 | 21 | 21 |
| GlucoTain^{®} liquiFlex (Clariant) Lauroyl/Myristoyl Methyl Glucamide (and) Coco-Betaine | 7.15 | 7.15 | 7.15 | 7.15 |
| GlucoTain^{®} Sense (Clariant) Sunfloweroyl Methyl Glucamide | 2.10 | 2.10 | 2.10 | 2.10 |
| Allantoin | 0.1 | 0.1 | 0.1 | 0.1 |
| Trisodium Citrate | 0.5 | 0.5 | 0.5 | 0.5 |
| Benzophenone-4 | 0.2 | 0.2 | 0.2 | 0.2 |
| Sodium Benzoate | 0.45 | 0.45 | 0.45 | 0.45 |
| Fragrance | 0.2 | 0.2 | 0.2 | 0.2 |
| Sodium Chloride | 2.0 | 2.0 | 2.0 | 2.0 |
| Citric Acid | As needed, pH 4.5 - 6.5 | | | |
| Sodium Hydroxide | As needed, pH 4.5 - 6.5 | | | |
| Colorant | As needed | | | |

### Example 22

### Shampoo compositions comprising the following ingredients:

| **Example** | **22a** | **22b** | **22c** | **22d** |
|---|---|---|---|---|
| Ingredient | wt% | wt% | wt% | wt% |
| Aqua | To 100 | To 100 | To 100 | To 100 |
| Opacifier composition according to Example 1 | 1.0 | - | - | - |
| Opacifier composition according to Example 2 | - | 1.5 | - | - |
| Opacifier composition according to Example 3 | - | - | 1.0 | - |
| Opacifier composition according to Example 4 | - | - | - | 1.0 |
| Glycerin | 1.0 | 1.0 | 1.0 | 1.0 |
| Polyquaternium-10 | 0.3 | 0.3 | 0.3 | 0.3 |
| Hydroxypropyl Guar (and) Hydroxypropyl Guar Hydroxypropyltrimonium Chloride | 0.15 | 0.15 | 0.15 | 0.15 |
| GlucoTain^{®} Care (Clariant) Cocoyl Methyl Glucamide | 7.5 | 7.5 | 7.5 | 7.5 |
| Hostapon^{®} SCI 85C (Clariant) Sodium Cocoyl Isethionate | 2.3 | 2.3 | 2.3 | 2.3 |
| Cocoamidopropyl Betaine | 16.7 | 16.7 | 16.7 | 16.7 |
| Nipaguard^{®} SCE (Clariant) Sorbitan Caprylate (and) Propanediol, Benzoic Acid | 1.2 | 1.2 | 1.2 | 1.2 |
| Panthenol | 0.5 | 0.5 | 0.5 | 0.5 |
| HerbEx^{™} Green Tea Butylene Glycol, Water, Camellia Sinensis Leaf Extract | 0.5 | 0.5 | 0.5 | 0.5 |
| Citric Acid | As needed, pH 4.5 - 6.5 | | | |
| Sodium Hydroxide | As needed, pH 4.5 - 6.5 | | | |
| Colorant | As needed | | | |

### Example 23

### Cleansing compositions (baby hair & body wash) comprising the following ingredients:

| **Example** | **23a** | **23b** | **23c** | **23d** |
|---|---|---|---|---|
| Ingredient | wt% | wt% | wt% | wt% |
| Aqua | To 100 | To 100 | To 100 | To 100 |
| Opacifier composition according to Example 5 | 1.7 | - | - | - |
| Opacifier composition according to Example 6 | - | 2.0 | - | - |
| Opacifier composition according to Example 9 | - | - | 2.2 | - |
| Opacifier composition according to Example 10 | - | - | - | 1.5 |
| Tetrasodium EDTA | 0.2 | 0.2 | 0.2 | 0.2 |
| Polyquaternium-43 | 0.3 | 0.3 | 0.3 | 0.3 |
| Genagen^{™} KB (Clariant) Coco-Betaine | 11 | 11 | 11 | 11 |
| GlucoTain^{®} Care (Clariant) Cocoyl Methyl Glucamide | 7.5 | 7.5 | 7.5 | 7.5 |
| Hostapon^{®} CCG (Clariant) Sodium Cocoyl Glutamate | 6 | 6 | 6 | 6 |
| GlucoTain^{®} Plus (Clariant) Capryloyl/Caproyl Methyl Glucamide (and) Lauroyl/Myristoyl Methyl Glucamide | 3 | 3 | 3 | 3 |
| Nipaguard^{®} SCE (Clariant) Sorbitan Caprylate (and) Propanediol, Benzoic Acid | 1 | 1 | 1 | 1 |
| Genapol^{®} LT (Clariant) PEG-150 Polyglyceryl-2 Tristearate (and) Laureth-3 (and) Glycol Distearate | 4 | 4 | 4 | 4 |
| Crambe Abyssinica Seed Oil | 1.5 | 1.5 | 1.5 | 1.5 |
| Citric Acid | As needed, pH 4.5 - 6.5 | | | |
| Sodium Hydroxide | As needed, pH 4.5 - 6.5 | | | |
| Colorant | As needed | | | |

## Claims

1. Opacifier composition comprising
(a) from 20 to 35 wt.-% of wax particles, based on the total weight of the opacifier composition; and
(b) from 2 to 12 wt.-% of a sulfate-free anionic surfactant, based on the total weight of the opacifier composition,
wherein the wax particles comprise mono- or di- C8-C20 esters of ethylene glycol, diethylene glycol or triethylene glycol, or rice bran wax, oxidized rice bran wax, rice bran wax ethyl esters, or mixtures thereof,
wherein the sulfate-free anionic surfactant is selected from acyl isethionates, acyl taurates, acyl glutamates, and mixtures thereof.

2. Opacifier composition according to claim 1, wherein the opacifier composition further comprises
(c) an amphoteric or zwitterionic surfactant.

3. Opacifier composition according to claim 1 or 2, wherein the opacifier composition further comprises
(d) a nonionic surfactant.

4. Opacifier composition according to any of the preceding claims, wherein the opacifier composition comprises
from 20 to 30 wt.-%, particularly preferably from 25 to 30 wt.-% of wax particles (a), based on the total weight of the opacifier composition; and
from 2 to 10 wt.-%, particularly preferably from 3 to 10 wt.-% of the sulfate-free anionic surfactant (b), based on the total weight of the opacifier composition.

5. Opacifier composition according to any of claims 2 to 4, wherein the opacifier composition comprises from 0.1 to 4.5 wt.-%, preferably from 0.2 to 3 wt.-%, particularly preferably from 0.3 to 2 wt.-% of the amphoteric or zwitterionic surfactant (c), based on the total weight of the opacifier composition.

6. Opacifier composition according to any of claims 3 to 5, wherein the opacifier composition comprises from 0.1 to 5 wt.-%, preferably from 0.3 to 3.5 wt.-%, particularly preferably from 0.5 to 2.5 wt.-% of the nonionic surfactant (d), based on the total weight of the opacifier composition.

7. Opacifier composition according to any of the preceding claims, wherein the wax particles comprise mono- or di- C12-C18 esters of ethylene glycol, or mixtures thereof.

8. Opacifier composition according to any of the preceding claims, wherein the sulfate-free anionic surfactant is selected from acyl isethionates, acyl taurates, and mixtures thereof.

9. Opacifier composition according to any of claims 2 to 8, wherein the amphoteric or zwitterionic surfactant comprises a betaine surfactant, preferably a C8- to C18-fatty acid alkylamidobetaine, particularly preferably cocamidopropyl betaine.

10. Opacifier composition according to any of claims 3 to 9, wherein the nonionic surfactant is selected from glyceryl fatty acid esters, polyglyceryl fatty acid esters, N-methyl-N-acylglucamines, and mixtures thereof, preferably is selected from esters of glycerol and one or more C8-C20 fatty acids, esters of polyglycerol having 2 to 20 glyceryl units and one or more C8-C20 fatty acids, and mixtures thereof.

11. Opacifier composition according to any of the preceding claims, wherein the opacifier composition is ethylene oxide-free.

12. Opacifier composition according to any of the preceding claims, wherein the opacifier composition of the invention has a renewable carbon index (RCI) of at least 90%, preferably at least 95%, particularly preferably at least 96%.

13. Use of a composition as defined in any of claims 1 to 12 as an opacifier in a cosmetic composition.

14. Cosmetic composition comprising an opacifier composition as defined in any of claims 1 to 12.

15. Cosmetic composition according to claim 14, wherein the cosmetic composition comprises from 0.5 to 5 wt.-%, preferably from 1 to 3 wt.-%, particularly preferably from 1 to 2 wt.-% of the opacifier composition, based on the total weight of the cosmetic composition.

## Patentansprüche

1. Trübungsmittelzusammensetzung, umfassend
(a) 20 bis 35 Gew.-% Wachspartikel, bezogen auf das Gesamtgewicht der Trübungsmittelzusammensetzung; und
(b) 2 bis 12 Gew.-% eines sulfatfreien anionischen Tensids, bezogen auf das Gesamtgewicht der Trübungsmittelzusammensetzung,
wobei die Wachspartikel Mono- oder Di-C8-C20-Ester von Ethylenglykol, Diethylenglykol oder Triethylenglykol oder Reiskleiewachs, oxidiertes Reiskleiewachs, Reiskleiewachs-Ethylester oder Mischungen davon umfassen, wobei das sulfatfreie anionische Tensid aus Acylisethionaten, Acyltauraten, Acylglutamaten und Mischungen davon ausgewählt ist.

2. Trübungsmittelzusammensetzung nach Anspruch 1, wobei die Trübungsmittelzusammensetzung ferner umfasst
(c) ein amphoteres oder zwitterionisches Tensid.

3. Trübungsmittelzusammensetzung nach Anspruch 1 oder 2, wobei die Trübungsmittelzusammensetzung ferner umfasst
(d) ein nichtionisches Tensid.

4. Trübungsmittelzusammensetzung nach einem der vorstehenden Ansprüche, wobei die Trübungsmittelzusammensetzung umfasst
20 bis 30 Gew.-%, besonders bevorzugt 25 bis 30 Gew.-%, an Wachspartikeln (a), bezogen auf das Gesamtgewicht der Trübungsmittelzusammensetzung; und
2 bis 10 Gew.-%, besonders bevorzugt 3 bis 10 Gew.-% des sulfatfreien anionischen Tensids (b), bezogen auf das Gesamtgewicht der Trübungsmittelzusammensetzung.

5. Trübungsmittelzusammensetzung nach einem der Ansprüche 2 bis 4, wobei die Trübungsmittelzusammensetzung 0,1 bis 4,5 Gew.-%, vorzugsweise 0,2 bis 3 Gew.-%, besonders bevorzugt 0,3 bis 2 Gew.-% des amphoteren oder zwitterionischen Tensids (c), bezogen auf das Gesamtgewicht der Trübungsmittelzusammensetzung, umfasst.

6. Trübungsmittelzusammensetzung gemäß einem der Ansprüche 3 bis 5, wobei die Trübungsmittelzusammensetzung 0,1 bis 5 Gew.-%, vorzugsweise 0,3 bis 3,5 Gew.-%, besonders bevorzugt 0,5 bis 2,5 Gew.-% des nichtionischen Tensids (d), bezogen auf das Gesamtgewicht der Trübungsmittelzusammensetzung, umfasst.

7. Trübungsmittelzusammensetzung nach einem der vorstehenden Ansprüche, wobei die Wachspartikel Mono- oder Di-C12-C18-Ester von Ethylenglykol oder Mischungen davon umfassen.

8. Trübungsmittelzusammensetzung nach einem der vorstehenden Ansprüche, wobei das sulfatfreie anionische Tensid aus Acylisethionaten, Acyltauraten und Mischungen davon ausgewählt ist.

9. Trübungsmittelzusammensetzung nach einem der Ansprüche 2 bis 8, wobei das amphotere oder zwitterionische Tensid ein Betain-Tensid umfasst, vorzugsweise ein C8- bis C18-Fettsäurealkylamidobetain, besonders bevorzugt Cocamidopropylbetain.

10. Trübungsmittelzusammensetzung nach einem der Ansprüche 3 bis 9, wobei das nichtionische Tensid ausgewählt ist aus Glycerinfettsäureestern, Polyglycerinfettsäureestern, N-Methyl-N-acylglucamine und Mischungen davon ausgewählt ist, vorzugsweise aus Estern von Glycerin und einer oder mehreren C8-C20-Fettsäuren, Estern von Polyglycerin mit 2 bis 20 Glycerineinheiten und einer oder mehreren C8-C20-Fettsäuren sowie Mischungen davon.

11. Trübungsmittelzusammensetzung gemäß einem der vorstehenden Ansprüche, wobei die Trübungsmittelzusammensetzung ethylenoxidfrei ist.

12. Trübungsmittelzusammensetzung nach einem der vorstehenden Ansprüche, wobei die Trübungsmittelzusammensetzung der Erfindung einen Renewable Carbon Index (RCI) von mindestens 90 %, vorzugsweise mindestens 95 %, besonders bevorzugt mindestens 96 % aufweist.

13. Verwendung einer Zusammensetzung gemäß einem der Ansprüche 1 bis 12 als Trübungsmittel in einer kosmetischen Zusammensetzung.

14. Kosmetische Zusammensetzung, die eine Trübungsmittelzusammensetzung gemäß einem der Ansprüche 1 bis 12 enthält.

15. Kosmetische Zusammensetzung nach Anspruch 14, wobei die kosmetische Zusammensetzung 0,5 bis 5 Gew.-%, vorzugsweise 1 bis 3 Gew.-%, besonders bevorzugt 1 bis 2 Gew.-% der Trübungsmittelzusammensetzung, bezogen auf das Gesamtgewicht der kosmetischen Zusammensetzung, enthält.

## Revendications

1. Composition opacifiante comprenant
(a) de 20 à 35 % en poids de particules de cire, par rapport au poids total de la composition opacifiante ; et
(b) de 2 à 12 % en poids d'un tensioactif anionique sans sulfate, par rapport au poids total de la composition opacifiante,
dans laquelle les particules de cire comprennent des esters mono- ou di- en C8-C20 d'éthylèneglycol, de diéthylèneglycol ou de triéthylèneglycol, ou de la cire de son de riz, de la cire de son de riz oxydée, des esters éthyliques de cire de son de riz, ou des mélanges de ceux-ci,
dans laquelle l'agent tensioactif anionique sans sulfate est choisi parmi les iséthionates d'acyle, les taurates d'acyle, les glutamates d'acyle et leurs mélanges.

2. Composition opacifiante selon la revendication 1, dans laquelle la composition opacifiante comprend en outre
(c) un tensioactif amphotère ou zwitterionique.

3. Composition opacifiante selon la revendication 1 ou 2, dans laquelle la composition opacifiante comprend en outre
(d) un tensioactif non ionique.

4. Composition opacifiante selon l'une quelconque des revendications précédentes, **caractérisée en ce que** la composition opacifiante comprend
de 20 à 30 % en poids, de préférence de 25 à 30 % en poids, de particules de cire (a), par rapport au poids total de la composition opacifiante ; et
de 2 à 10 % en poids, de préférence de 3 à 10 % en poids, d'agent tensioactif anionique sans sulfate (b), par rapport au poids total de la composition opacifiante.

5. Composition opacifiante selon l'une quelconque des revendications 2 à 4, dans laquelle la composition opacifiante comprend de 0,1 à 4,5 % en poids, de préférence de 0,2 à 3 % en poids, de manière particulièrement préférée de 0,3 à 2 % en poids de tensioactif amphotère ou zwitterionique (c), par rapport au poids total de la composition opacifiante.

6. Composition opacifiante selon l'une quelconque des revendications 3 à 5, dans laquelle la composition opacifiante comprend de 0,1 à 5 % en poids, de préférence de 0,3 à 3,5 % en poids, et de manière particulièrement préférée de 0,5 à 2,5 % en poids de l'agent tensioactif non ionique (d), par rapport au poids total de la composition opacifiante.

7. Composition opacifiante selon l'une quelconque des revendications précédentes, dans laquelle les particules de cire comprennent des esters mono- ou di- en C12-C18 de l'éthylène glycol, ou des mélanges de ceux-ci.

8. Composition opacifiante selon l'une quelconque des revendications précédentes, dans laquelle l'agent tensioactif anionique sans sulfate est choisi parmi les iséthionates d'acyle, les taurates d'acyle et leurs mélanges.

9. Composition opacifiante selon l'une quelconque des revendications 2 à 8, dans laquelle le tensioactif amphotère ou zwitterionique comprend un tensioactif de type bétaïne, de préférence une alkylamidobétaïne d'acide gras en C8 à C18, de manière particulièrement préférée la cocamidopropylbétaïne.

10. Composition opacifiante selon l'une quelconque des revendications 3 à 9, dans laquelle le tensioactif non ionique est choisi parmi les esters d'acides gras de glycéryle, les esters d'acides gras de polyglycéryle, des N-méthyl-N-acylglucamines et de leurs mélanges, de préférence parmi les esters de glycérol et d'un ou plusieurs acides gras en C8 à C20, les esters de polyglycérol comportant de 2 à 20 unités de glycéryle et d'un ou plusieurs acides gras en C8 à C20, et leurs mélanges.

11. Composition opacifiante selon l'une quelconque des revendications précédentes, dans laquelle la composition opacifiante est exempte d'oxyde d'éthylène.

12. Composition opacifiante selon l'une quelconque des revendications précédentes, dans laquelle la composition opacifiante de l'invention présente un indice de carbone renouvelable (RCI) d'au moins 90 %, de préférence d'au moins 95 %, et de manière particulièrement préférée d'au moins 96 %.

13. Utilisation d'une composition telle que définie dans l'une quelconque des revendications 1 à 12 en tant qu'opacifiant dans une composition cosmétique.

14. Composition cosmétique comprenant une composition opacifiante telle que définie dans l'une quelconque des revendications 1 à 12.

15. Composition cosmétique selon la revendication 14, dans laquelle la composition cosmétique comprend de 0,5 à 5 % en poids, de préférence de 1 à 3 % en poids, et de manière particulièrement préférée de 1 à 2 % en poids de la composition opacifiante, par rapport au poids total de la composition cosmétique.
